# EUROPEAN PATENT SPECIFICATION

(11) **EP 0 869 986 B1**
(45) Date of publication and mention of the grant of the patent: **02.11.2000**
(21) Application number: 97901352.1
(22) Date of filing: 03.01.1997
(51) Int. Cl.: C08G 65/34, C08G 63/664, A61K 9/22, A61L 27/00

(54) **POLYMERS WITH CONTROLLED PHYSICAL STATE AND BIOERODIBILITY**
POLYMERE MIT KONTROLLIERTEM PHYSIKALISCHEN ZUSTAND UND BIOERODIERBARKEIT
POLYMERES A ETAT PHYSIQUE ET ERODABILITE MAITRISES

(30) Priority: 05.01.1996 US 583585; 05.01.1996 US 583649
(43) Date of publication of application: 14.10.1998
(73) Proprietor: Advanced Polymer Systems, Inc., Redwood City, CA 94063 (US)
(72) Inventor: HELLER, Jorge, Woodside, CA 94062 (US); NG, Steve, Y., San Francisco, CA 94122 (US)
(74) Representative: Harrison, David Christopher
(86) International application number: US9700144
(87) International publication number: WO9725366

(56) References cited:
- EP-A- 0 419 156
- WO-A-91/03510

## Description

### BACKGROUND OF THE INVENTION

### A. Field of the Invention

The present invention relates to polymers which have pharmaceutical, cosmetic and clinical applications. In particular, this invention relates to polymers whose mechano-physical state and the rate at which they erode in a biological environment are controllable in degrees by their molecular structures.

### B. Description of the Prior Art

Interest in synthetic biodegradable polymers for the systemic delivery of therapeutic agents began in the early 1970's with the work of Yolles *et al., Polymer News* **1**:9-15 (1970) using poly(lactic acid). Since that time, numerous other polymers have been prepared and investigated as bioerodible matrices for the controlled release of therapeutic agents.

U.S. Patent Nos. 4,079,038, 4,093,709, 4,131,648, 4,138,344 and 4,180,646 disclose biodegradable or bioerodible poly(ortho ester)s. These polymers are formed by a reaction between an ortho ester (or orthocarbonate) such as 2,2-diethoxytetrahydrofuran and a diol such as 1,4-cyclohexanedimethanol. The reaction requires elevated temperature and reduced pressure and a relatively long reaction time. Drugs or other active agents are retained in the polymer matrix to be released as the polymer biodegrades due to hydrolysis of the labile linkages.

U.S. Patent No. 4,304,767 discloses polymers prepared by reacting a polyol with a polyfunctional ketene acetal. These polymers represent a significant improvement over those of US-A-4,079,038, 4,093,709, 4,131,648, 4,138,344 and 4,180,646, since synthesis proceeds readily at room temperature and atmospheric pressure, and the resulting polymers have superior properties.

Further polymers are disclosed in US-A 4,957,998. These polymers contain acetal, carboxy-acetal and carboxy-ortho ester linkages, and are prepared by a two-step process beginning with the reaction between a polyfunctional ketene acetal and a compound containing a vinyl ether, followed by reaction with a polyol or polyacid.

Still further polymers of a similar type are disclosed in US-A-4,946,931. The polymers are formed by a reaction between a compound containing a multiplicity of carboxylate functions and a polyfunctional ketene acetal. The resulting polymers have very rapid erosion times.

Despite the ease with which the ortho ester linkage hydrolyzes, poly(ortho ester)s known in the prior art are extremely stable materials when placed in an aqueous buffer, or when residing in the body. This stability is attributable to the extreme hydrophobicity of the poly(ortho ester)s which severely limits the amount of water that can penetrate the polymer. To achieve useful erosion rates, therefore, acidic excipients must be physically incorporated into the polymer. While this allows control over erosion rates, the physically incorporated acidic excipient can diffuse from the polymer matrix at varying rates, leaving a matrix that is completely depleted of excipient while the polymer still has a very long lifetime remaining.

EP-A-419156 discloses bioerodible thermoset elastomers formed by the reaction of diketons acetals with various polyols.

### SUMMARY OF THE INVENTION

It has now been discovered that polymers useful as orthopedic implants or vehicles for the sequestration and sustained delivery of drugs, cosmetic agents and other beneficial agents can be prepared in such a manner that the rate and degree to which they are hydrolyzed by contact with bodily fluids at the normal body temperature and pH, can be controlled without addition of exogeneous acid. This discovery resides in the incorporation of esters of short-chain α-hydroxy acids such as esters of glycolic acid, lactic acid or glycolic-co-lactic acid copolymer into the polymer chain and variation of the amount of these esters relative to the polymer as a whole.

In the presence of water, these esters, when incorporated into the polymer chain, are readily hydrolyzed at a body temperature of 37°C and a physiological pH, in particular at a pH of 7.4, to produce the corresponding α-hydroxy acids. The α-hydroxy acids then act as an acidic excipient to control the hydrolysis rate of the polymer. When the polymer is used as a vehicle or matrix entrapping an active agent, the hydrolysis of the polymer causes release of the active agent.

In addition, the mechano-physical state of the polymer may also be controlled. This is achieved by the inclusion of the residues of certain diols in selected proportions relative to the polymer as a whole. For example, a high content of the residue of *trans*-cyclohexanedimethanol relative to a "soft" diol (definition of which is given below) produces a relatively rigid polymer chain and a more solid substance, and by decreasing the *trans*-cyclohexanedimethanol content relative to the "soft" diol, the polymer will change progressively through the stages of a rigid thermoplastic, a soft thermoplastic, a low melting solid to an ointment-like (viscous liquid) material, and any stage in between.

The polymers of the present invention are prepared by condensation reactions between diketene acetals and polyols, preferably diols, which are or include α-hydroxy-acid-containing diols and the variation in mechano-physical state and rate of hydrolysis (bioerodibility) is achieved by the selection and use of combinations of different types of diols.

### BRIEF DESCRIPTION OF THE FIGURES

FIG. 1 is a reference plot showing the rate of bioerodibility of a polymer that does not contain a hydrolyzable ester moiety of an α-hydroxy acid.

FIG. 2 is a plot of the rate of bioerodibility of various polymers containing a glycolic acid ester moiety at four different levels.

FIG. 3 is a plot showing how variations in the amount of a glycolic acid ester moiety in a polymer affects the rate at which a sequestered active agent is released from the polymer.

### DETAILED DESCRIPTION OF THE INVENTION

### I. Definitions

Unless defined otherwise in this specification, all technical and scientific terms are used herein according to their conventional definitions as they are commonly used and understood by those of ordinary skill in the art of synthetic chemistry, pharmacology and cosmetology.

The term "matrix" denotes the physical structure of the polymer. Solid matrices essentially retain the active agent in a manner preventing release of the agent until the polymer erodes or decomposes.

The terms "vehicle" and "carrier" denote an ingredient that is included in a composition such as a pharmaceutical or cosmetic preparation for reasons other than a therapeutic or other biological effect. Functions served by vehicles and carriers include transporting an active agent to a site of interest, controlling the rate of access to, or release of, the active agent by sequestration or other means, and facilitating the application of the agent to the region where its activity is needed.

The terms "controlled release", "sustained release" and similar terms are used to denote a mode of active agent delivery that occurs when the active agent is released from the vehicle or carrier at an ascertainable and manipulatable rate over a period of time, rather than dispersed immediately upon ingestion or application. Controlled or sustained release may extend for hours, days or months, and may vary as a function of numerous factors. In the present invention, an important determinant of the rate of delivery is the rate of hydrolysis of the linkages between and within the units of the polymer. The rate of hydrolysis in turn may be controlled by the composition of the polymer and the number of hydrolyzable bonds in the polymer. Other factors include particle size, particle composition, particle hydration, acidity of the medium (either internal or external to the matrix), solubility of the active agent in the matrix and molecular weight and charge density of the active agent.

The term "active agent" is intended to include any compound or mixture of compounds which produces a beneficial or useful result. Active agents are distinguishable from such components as vehicles, carriers, diluents, lubricants, binders and other formulating aids, and encapsulating or otherwise protective components. Examples of active agents are pharmaceutical, agricultural or cosmetic agents. Suitable pharmaceutical agents include antigens, antibodies, vaccines, hormones (for example, estrogens, progestins, androgens, adrenocortical steroids, insulin, erythropoietin and the like), vitamins, enzymes, proteins, naturally occurring or bioengineered substances, anti-infectives (including antibiotics, antivirals, fungicides, scabicides or pediculicides), antipsychotic agents (for example, phenothiazines including chlorpromazine, triflupromazine, mesoridazine, piperacetazine and thioridazine; thioxanthenes including chlorprothixene; and the like), anti-anxiety agents (for example, benzodiazepines including diazepam, alprazolam, clonazepam, oxazepam; and barbiturates), anti-depressants (including tricyclic antidepressants and monoamine oxidase inhibitors including imipramine, amitriptyline, doxepin, nortriptyline, amoxapine, tranylcypromine, phenelzine and the like), stimulants (for example, methylphenidate, doxapram, nikethamide and the like), narcotics (for example, morphine, meperidine, codeine and the like), analgesic-antipyretics and anti-inflammatory agents (for example, aspirin, ibuprofen, naproxen and the like), local anesthetics (for example, procaine, lidocaine, tetracaine and the like), fertility control agents, chemotherapeutic and anti-neoplastic agents (fore example, mechlorethamine, cyclophosphamide, uracil, fluorouracil, thioguanine, carmustine, lomustine, melphalan, chlorambucil, streptozocin, methotrexate, vincristine, bleomycin, vinblastine, vindesine, dactinomycin, daunorubicin, doxorubicin, tamoxifen and the like), cardiovascular and antihypertensive agents (for example, procainamide, amyl nitrite, nitroglycerin, propranolol, metoprolol, prazosin, phentolamine, trimethaphan, captopril, enalapril and the like), drugs for the therapy of pulmonary disorders, anti-epilepsy agents (for example, phenytoin, ethotoin and the like), antipruritics, astringents, anti-hidrotics, keratolytic agents, keratoplastic agents, rubefacients, sunscreens, pigmentation agents or emollients. The term "active agents" further include pesticides, herbicides, plant growth promoters or inhibitors, preservatives, disinfectants, air purifiers and nutrients.

"Sequestration" is the confinement or retention of an active agent within the internal spaces of a polymer matrix. Sequestration of an active agent within the matrix may limit the toxic effect of the agent, prolong the time of action of the agent in a controlled manner, permit the release of the agent in a precisely defined location in an organism, or protect unstable agents against the action of the environment.

The term "unit" denotes an individual segment of a polymer chain, which, for the purpose of this invention, consists of the residue of a diketene acetal molecule and the residue of a polyol. The specific structure of a "unit" formed from a diketene acetal molecule with a diol is represented by Formula I(unit) in the following sections.

An "α-hydroxy acid containing" unit denotes an individual unit, the amount of which relative to the polymer as a whole determines the rate of hydrolysis (or bioerodibility) of the polymer, and in turn, the release rate of the active agent. The specific structure of an "α-hydroxy acid containing" unit is represented by Formula I(a) in the following sections.

The terms, "hard" and "soft" units denote individual units of polymer, the contents of which relative to the polymer as a whole determine the mechano-physical state of the polymer. The specific structures of "hard" and "soft" units are represented by Formulas I(b) and I(c) respectively in the following sections.

The term "polyol" refers to a chemical compound having more than one hydroxy (-OH) functional group.

The term "diol" refers to a chemical compound having two hydroxy (-OH) groups. For the purpose of this invention, the diols are classified into three groups: "a-hydroxy acid containing" diols, "hard" diols and "soft" diols. The specific structures of these three types of diols are represented by Formulas III(a), III(b) and III(c) respectively in the following sections.

The term "alkyl" denotes a branched or unbranched saturated hydrocarbon radical having from one to the number of carbon atoms designated (e.g., C₁-C₁₂ alkyl). Examples of alkyl include methyl, ethyl, n-propyl, isopropyl, n-butyl, t-butyl, n-hexyl, n-octyl and the like.

The term "alkylene" denotes a branched or unbranched saturated divalent radical having from one to the number of carbon atoms designated (e.g., C₁-C₁₂ alkylene). Examples of alkylene include methylene (-CH₂-), ethylene (-CH₂CH₂-), isopentylene (-CH₂-CH(CH₃)-CH₂-CH₂-), n-octylene (-(CH₂)₈-) and the like.

The terms "bioerodible" and "bioerodibility" refer to the degradation, disassembly or digestion of the polymer by action of a biological environment, including the action of living organisms and most notably at physiological pH and temperature. A principal mechanism for bioerosion of the polymers of the present invention is hydrolysis of linkages between and within the units of the polymer. In the present invention, the rate of hydrolysis of the linkages may be determined by the content of the "α-hydroxy acid containing" units relative to the polymer as a whole.

The terms "flexible" and "rigid" refer to the mechano-physical state of the polymer. A "flexible" chain generally contains a relative high amount of "soft" units and may impart a liquid or ointment-like quality, while a "rigid" chain generally contains a relative high amount of "hard" units and tends to impart a solid, plastic-like consistency.

The term "polymer hydrolysis", for the purpose of this invention, refers to the hydrolysis of the linkages between and within the units of the polymer.

The term "mole percent", for the purpose of this invention, refers to the number of a particular type of unit (e.g., the "α-hydroxy acid containing" unit, "hard" unit or "soft" unit) in 100 individual units of the polymer chain.

The term "molecular weight" refers to the sum of the weights of all atoms in the polymer chain.

### II. Structure of the Polymer

The polymer of the present invention is represented by the following general formula 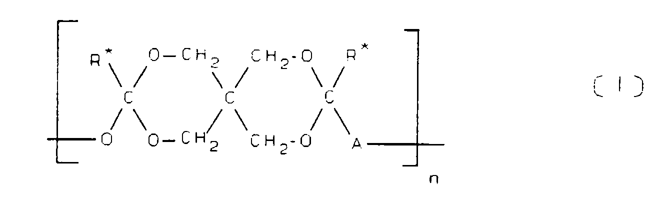 wherein A is -O-R¹-, -O-R²- or (-O-R³)_{q}-; the definitions of R¹, R², R³ and q are specifically given below; R* is a C₁-C₄ alkyl; and n is ≥5.

Each individual "unit" of the polymer represented by the following formula 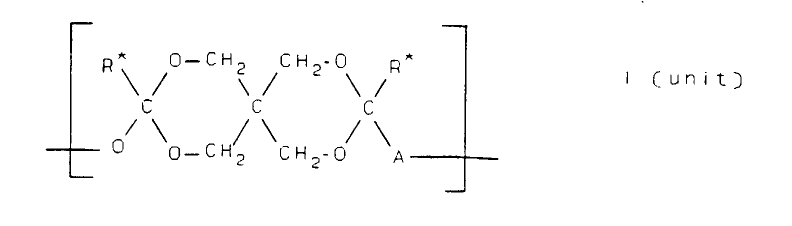 may be an "α-hydroxy acid containing" unit (in which A is -O-R¹-), a "hard" unit (in which A is -O-R²-) or a "soft" unit (in which A is (-O-R³)_{q}-) ; provided that the polymer contains at least 0.1 mole percent of "α-hydroxy acid containing" units.

An "α-hydroxy acid containing" unit is represented by the following formula 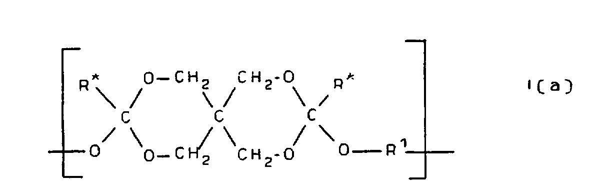 wherein R* is a C₁-C₄ alkyl and R¹ is 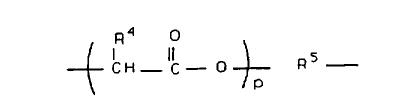 in which:
p is 1-10;
R⁴ is hydrogen or a C₁-C₆ alkyl; and
R⁵ is

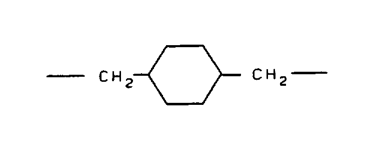

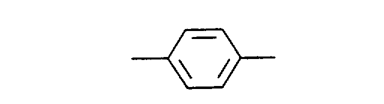

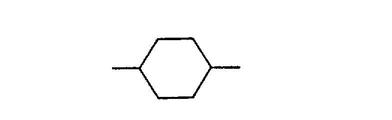

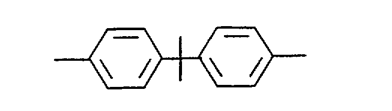

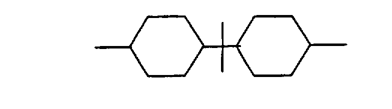

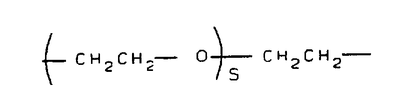

   (̵CH₂)̵ₜ

   or 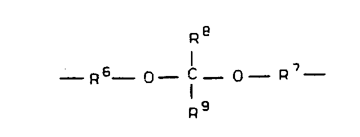
wherein:
s is 1 to 100;
t is 1 to 12;
R⁶ and R⁷ are independently a C₁-C₁₂ alkylene;
R⁸ is hydrogen or a C₁-C₆ alkyl; and
R⁹ is a C₁-C₆ alkyl; or
R⁸ and R⁹ taken together are a C₃-C₁₀ alkylene.

A "hard" unit is represented by the following formula 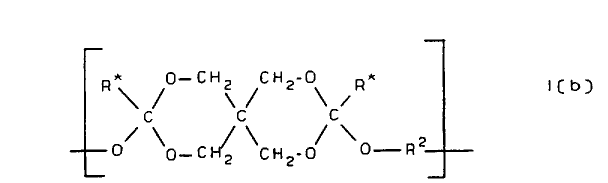 wherein R* is a C₁-C₄ alkyl and R² is 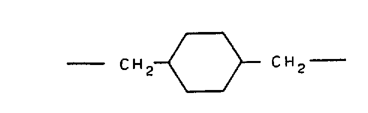 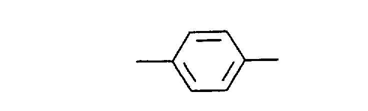 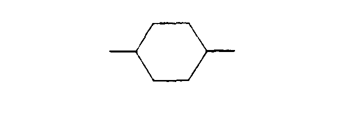 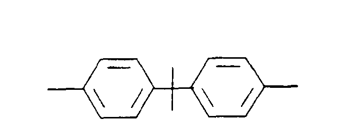 or

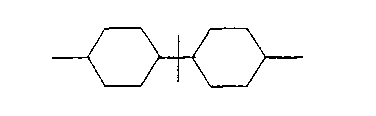

A "soft" unit is represented by the following formula 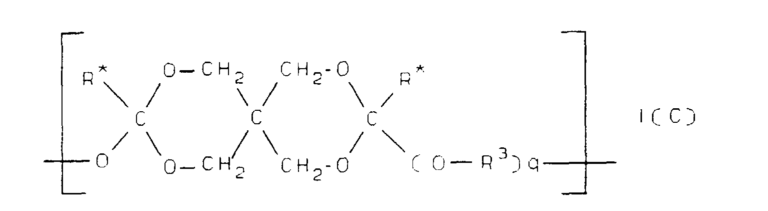 wherein R* is a C₁-C₄ alkyl; q is 1 to 20; and
when q is 1, R³ is 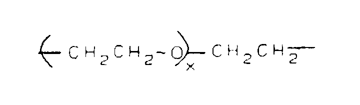 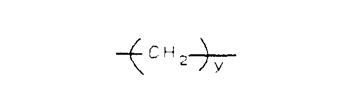 or 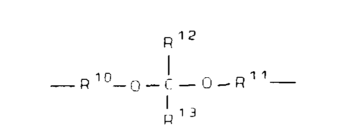 in which:
x is 1 to 100;
y is 1 to 12;
R¹⁰ and R¹¹ are independently a C₁-C₁₂ alkylene;
R¹² is hydrogen or a C₁-C₆ alkyl; and
R¹³ is a C₁-C₆ alkyl; or
R¹² and R¹³ taken together are a C₃-C₁₀ alkylene; and
when q is 2 to 20, each R³ may be the same or different and is selected from the group consisting of 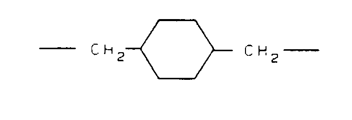 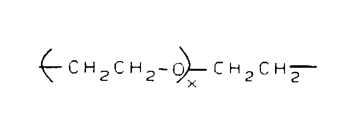 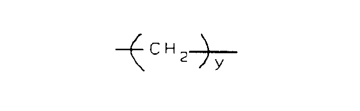 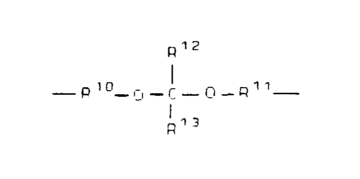 and 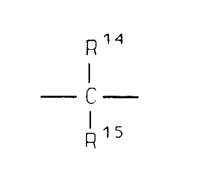 wherein x, y, R¹⁰, R¹¹, R¹² and R¹³ are as defined above, R¹⁴ is hydrogen or a C₁-C₄ alkyl, and R¹⁵ is a C₁-C₄ alkyl.

The structure of the polymer of this invention, as shown in general Formula I, is one of alternating residues of the diketene acetal and a diol, with each adjacent pair of diketene acetal residues being separated by the residue of one polyol, preferably a diol.

The rate of hydrolysis and mechano-physical state of the polymer are determined by the contents of the three types of units. Generally, the polymer may consist of 5 to 1000 individual units.

The composition of the polymer may also be conveniently expressed in mole percentage. The "α-hydroxy acid containing" unit constitutes about 0.1 to 100 mole percent of the polymer as a whole. Each of the "hard" and "soft" units constitutes about 0-99.9 mole percent of the polymer.

It is also understood that the present invention encompasses cross-linked polymers prepared from a diketene acetal and a mixture of polyols which comprises one or more polyols having more than two hydroxy functional groups.

### III. Preferred Embodiments

The preferred embodiment of the present invention is the polymer of Formula (I) wherein n is about 5 to about 1000, more preferably about 20 to about 500, and most preferably about 30 to about 300. The molecular weight of the polymer consequently ranges from about 1,000 to about 500,000.

Expressed in terms of mole percent of the "hard" unit relative to the polymer as a whole, preferred polymers for liquid or ointment-like compositions are those in which the "hard" unit constitutes about 20 mole percent or less. Likewise, preferred polymers for more solid compositions are those in which the "hard" unit constitutes from about 60 mole percent to about 99.9 mole percent.

Polymers having a higher content of the "α-hydroxy acid containing" unit will have a higher rate of bioerodibility. Preferred polymers are those in which the "α-hydroxy acid containing" units constitute preferably from about 1 to about 50 mole percent, and more preferably from about 2 to about 30 mole percent.

With respect to the individual "α-hydroxy acid containing" unit of Formula I(a) wherein R¹ is 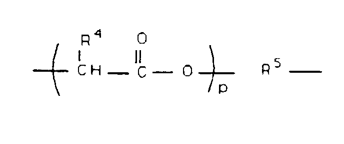 p is preferably 1 to 6, more preferably 1 to 4, most preferably 1 or 2; R⁴ is preferably hydrogen or methyl; and in the above definitions of R⁵, s is preferably 2 to 12, more preferably 2 to 6 and most preferably 2; t is preferably 4 to 12, more preferably 4 to 6 and most preferably 6; R⁶ and R⁷ are preferably identical, more preferably an unbranched C₄-C₁₂ alkylene and most preferably an unbranched C₆-C₁₂ alkylene; R⁸ is preferably hydrogen; and R⁹ is preferably methyl.

With respect to the individual "hard" unit of Formula I(b), R² is preferably

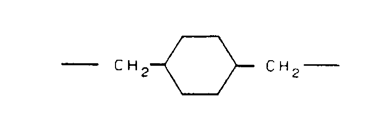

With respect to the individual "soft" unit of Formula I(c), q is preferably 1 to 6 and more preferably 1 to 3; and in the definitions of R³, x is preferably 2 to 12, more preferably 2 to 6 and most preferably 2; y is preferably 4 to 12, more preferably 4 to 6 and most preferably 6; R¹⁰ and R¹¹ are preferably identical, more preferably an unbranched C₄-C₁₂ alkylene and most preferably an unbranched C₆-C₁₂ alkylene; R¹² is preferably hydrogen; R¹³ is preferably methyl; R¹⁴ is preferably hydrogen and R¹⁵ is preferably methyl.

Further preferred embodiments are those in which the diol mixture contains one of the various specific diols listed in Example 2, as well as various combinations of diols used in Examples 3 and 4.

### IV. Preparation of the Polymer

The polymer of this invention is prepared by the reaction of a diketene acetal of Formula II 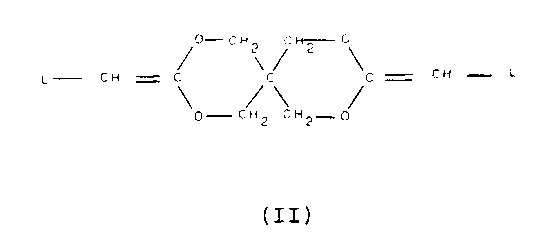 wherein L is hydrogen or a C₁₋₃ alkyl, with a diol of Formula III(a), or a mixture of two or three diols of Formulas III(a)-III(c) in selected proportions, with the proviso that at least 0.1% of the totol diol mixture is the diol of Formula III(a):

HO-R¹-OH III(a)

HO-R²-OH III(b)

H(-O-R³)_{q}-OH III(c)

wherein q, R¹, R² and R³ are as defined above.

The "α-hydroxy acid containing" unit of Formula I(a) is formed by the reaction between the diketene acetal of Formula II with a diol of Formula III(a).

Similarly, the "hard" unit of Formula I(b) is formed by the reaction between the diketene acetal of Formula II with a diol of Formula III(b), and the "soft" unit of Formula I(c) is formed by the reaction between the diketene acetal of Formula II with a diol of Formula III(c).

To form the polymer using diols of the three types, a diol mixture is formed with selected proportions based on the desired characteristics of the polymer. The diol mixture contains at least 0.1 mole percent of the diol of Formula III(a).

The invention includes polymers which are prepared from a mixture of the three types of diols as well as polymers prepared from only the diol of Formula III(a) or a mixture of two of the three types of diols, one of which is the diol of Formula III(a). It also includes polymers prepared from a mixture of diols which contains two or more diols of the same type.

The preparation of the diketene acetal of Formula II is disclosed in United States Patent Nos. 4,304,767 and 4,532,335. The diols are prepared according to methods known in the art. Some of the diols suitable for the present invention are also commercially available. For example, *trans*-cyclohexane-dimethanol can be purchased from Cros-Organics (New Jersey). The preparation of diols, in particular the "soft" diols of Formula III(c) is generally disclosed in Heller *et al., J. Polymer Sci., Polymer Letters Ed.* **18**:293-297 (1980), by reacting an appropriate divinyl ether with an appropriate diol. The "α-hydroxy acid containing" diol of Formula III(a) that comprises a polyester moiety may be prepared by reacting a selected diol (which is expressed as HO-R⁵-OH) with between 0.5 and 5 molar equivalents of a cyclic ester of an α-hydroxy acid, and allowing the reaction to proceed at 100-200°C for a period of time ranging from about 12 hours to about 48 hours. Although particular solvents are not required for this reaction, organic solvents such as dimethylacetamide, dimethyl sulfoxide, dimethylformamide, acetonitrile, pyrrolidone, tetrahydrofuran, and methylbutyl ether may be used.

Once made, the "α-hydroxy acid containing" diol of Formula III(a) and the diols of Formulas III(b) and/or III(c) with selected proportions are mixed with the diketene acetal of Formula II, in accordance with the 1:1 stoichiometric ratio of total number of moles of diketene acetal to total number of moles of diols, in a suitable solvent at ambient temperature. The condensation reaction between the diketene acetal and the diols is carried out under conditions which are well known to those skilled in the art and will also be readily apparent from the structures of the reactants themselves. Suitable solvents are polar aprotic solvents, such as dimethylacetamide, dimethyl sulfoxide, dimethylformamide, acetonitrile, pyrrolidone, tetrahydrofuran, and methylbutyl ether, and the like. Catalysts are not required for this reaction, but when used, suitable catalysts are iodine in pyridine, *p*-toluenesulfonic acid; Lewis acids (such as boron trichloride, boron trifluoride, boron trichloride etherate, boron trifluoride etherate, stannic oxychloride, phosphorous oxychloride, zinc chloride, phosphorous pentachloride, antimony pentafluoride, stannous octoate, stannic chloride, diethyl zinc, and mixtures thereof); and Brönsted catalysts (such as polyphosphoric acid, crosslinked polystyrene sulfonic acid, acidic silica gel, and mixtures thereof). A typical amount of catalyst used is about 0.2% by weight relative to the diketene acetal. Smaller or larger amounts can also be used, such as 0.005% to about 2.0% by weight relative to the diketene acetal of Formula II.

Once the reaction is complete, the reaction mixture is allowed to cool to room temperature. About ten volumes of a precipitant such as anhydrous methanol or hexane at room temperature are then added, and the precipitated polymer may be collected by filtration or decanting, and dried in a vacuum oven at 30-40°C.

The rigidity or flexibility of the polymer is determined by the proportions of the "hard" and "soft" units in the polymer structure, with greater rigidity achieved by including greater proportions of the "hard" diol of Formula III(b) in the diol mixture.

The bioerodibility of the polymer is determined by the proportion of the hydrolyzable α-hydroxy acid ester groups, with greater bioerodibility achieved by including greater proportions of the "α-hydroxy acid containing" diol of Formula III(a) in the diol mixture.

Thus, both characteristics of the resulting polymer prepared from the reaction between the diketene acetal of Formula II and a mixture of the diols, are controlled by the ratio of quantities of the three types of diols in the diol mixture.

It is also understood that the present invention encompasses cross-linked polymers which are prepared by employing one or more polyols having more than two hydroxy functional groups. Such cross-linked polymers may be prepared preferably by first reacting the diketene acetal with a diol of Formula III(a) optionally with a diol of Formula III(b) and/or a diol of Formula III(c) followed by addition of the polyol(s) having more than two hydroxy functional groups. Alternatively, the polyol(s) having more than two hydroxy functional groups may be added simultaneously with the diol of Formula III(a) and any other diols. Polyols having more than two hydroxy functional groups suitable for the preparation of the cross-linked polymers may be the straight or branched chain type, including polyhydroxyl compounds such as 1,2,3-propanetriol, 1,2,5-pentanetriol, 1,2,6-hexanetriol, 1,3,5-pentanetriol, 1,2,4-butanetriol, 1,4,7-heptanetriol, 1,5,10-decanetriol, 1,5,12-dodecanetriol, 1,2,3,4,5,6-hexanehexol and the like. Other representative polyols of the type are described in US Patent No. 4,304,767. The reaction conditions (e.g., suitable solvents and reaction temperatures) and procedures for the preparation of the cross-linked polymers are similar to those described above for the preparation of the polymers employing only the diols, and are also described in US Patent No. 4,304,767.

### V. Use of the Polymer

The present polymers can be used as orthopedic implants or as vehicles for the sustained release of an active agent. To use a polymer in this manner, the polymer is first mixed with the active agent. High homogeneity may be achieved by mixing the polymer in its heat softened state with the active agent, followed by lowering the temperature to harden the composition. Alternatively, the polymer can be dissolved in an appropriate casting solvent, such as tetrahydrofuran, methylene chloride, chloroform or ethyl acetate, and the active agent can then be dispersed or dissolved in the polymer solution, followed by evaporating the solvent to achieve the finished composition. Another method is grinding a solid polymer material into powder which is then mixed with a powdered active agent. The active agent may also be incorporated into the mixture of monomers before polymerization.

The composition obtained from either one of the above methods can be readily processed into a variety of shapes and forms for implantation, insertion or placement on the body or into body cavities or passageways. For example, the polymer composition may be injection molded, extruded or compressed into a thin film or made into devices of various geometric shapes or forms such as flat, square, round, cylindrical, tubular, disc, ring and the like.

The polymer composition may also be injected by syringe subcutaneously or intramuscularly as particles of 0.1µ to 1000µ, preferably 0.5µ to 200µ, and more preferably 1µ to 150µ suspended in a pharmaceutically acceptable injection base. Liquid vehicles useful for suspending the drug-polymer composition for injection include isotonic saline solution or oils (such as corn oil, cottonseed oil, peanut oil and sesame oil) which, if desired, may contain other adjuvants.

Another injectable dosage form may be prepared from an active agent mixed in with a polymer of the present invention which has an ointment-like consistency. Such a dosage form may be administered by injection with or without a solvent.

The polymer composition administered by either injection or implantation undergoes bioerosion in the body into non-toxic and non-reactive materials. By controlling the number of hydrolyzable bonds in the polymer, the active agent may be released at a desired rate. The implants prepared from the present polymers, in which the polymer constitutes the matrix containing an active agent, also have the advantage that they do not require removal, because of the bioerodible nature of the polymer.

In some cases, particles with cores of the pure active agent coated with various thicknesses of the present polymer may be preferred for sustained delivery of the active agent. Coating of the discrete drug particles may be accomplished by conventional methods which are all well-known to the person skilled in the art. For example, finely divided drug particles may be suspended in a solvent system (in which the drug is not soluble) containing the dissolved polymer and other excipients, followed by spray drying. Alternatively, the drug particles may be placed in a rotating pan or a fluid-bed dryer and the polymer dissolved in a carrier solvent is sprayed onto the drug particles until a suitable coating quantity is deposited on the particles to give a desired thickness. The coating may also be achieved by suspending the drug particles in a solvent system containing the dissolved polymer followed by adding to the suspension a non-solvent causing the polymer to precipitate and form a coating over the drug particles.

For the sustained release compositions, because the active agent will be released over a controlled period of time, the agent usually is present in an amount which is greater than the conventional single dose. The relative proportions of the active agent and the polymer can vary over a wide range (e.g., 0.1 to 50 weight percent) depending on the therapeutic agent and the desired effect.

Sustained compositions of cosmetic and agricultural agents may also be prepared by any one of the methods as described above, using the polymers of the present invention.

The solid polymers (those containing a high percentage of the "hard" unit) are also useful for a variety of orthopedic applications. For example, they can be used as fracture fixation devices for repair of osteochondral defects, ligament and tendon reconstructions and bone substitutes. In addition, the fact that the present polymers permit simultaneous selection of both a desired level of their mechano-physical state and a desired rate of bioerodibility, also renders them attractive as grafts or scaffolds on which cells can be cultured *in vitro* prior to implantation to regenerate tissues. Tissues which can be regenerated using this approach include but not limited to, bone, tendon, cartilage, ligaments, liver, intestine, ureter and skin tissues. For example, the polymers may be used to regenerate skin for patients with burns or skin ulcers. Cartilages may be repaired by first isolating chondrocytes from a patient (or a donor), allowing them to proliferate on the scaffolds prepared from the present polymer and re-implanting the cells in the patient.

The polymer scaffolds or implants may further contain other biologically active substances or synthetic inorganic materials such as re-enforcing filler material for enhancing the mechanic property of the scaffolds or implants (e.g. calcium sodium metaphosphate fibers), antibiotics or bone growth factors to induce and/or promote orthopedic restoration and tissue regeneration.

### EXAMPLES

### Example 1

### Preparation of Diketene Acetal of Formula (II)

### Synthesis of 3,9-di(ethylidine)-2,4,8,10-tetraoxaspiro[5,5]undecane (DETOSU)

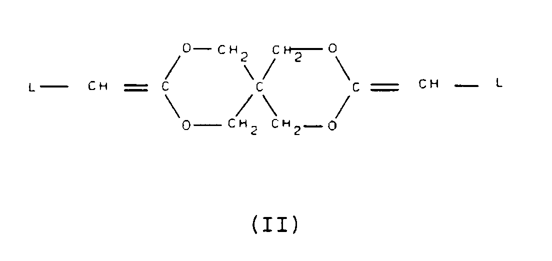

A 3-liter, 3-necked flask fitted with a mechanical stirrer, argon inlet tube, thermometer and rubber septum was charged with 1.2 L of ethylene diamine. The flask was cooled with ice water and the contents kept at about 8°C under an argon atmosphere. A hexane solution of n-butyllithium (130 g, 2 moles) was added via a stainless steel hypodermic U-tube pushed through the rubber septum using carefully controlled argon pressure over a period of 1 hour. Next, a mixture of 3,9-divinyl-2,4,8,10-tetraoxaspiro[5,5]undecane (available from Aldrich Chemical Company, Inc., Milwaukee, Wisconsin, USA) (530 g, 2.5 moles) and 0.5 L of ethylenediamine was cooled to 8°C and added to the 3-necked flask. After stirring at 8°C for 3 hours, the reaction mixture was poured into 3 L of ice water with vigorous stirring. The aqueous mixture was extracted twice with 1-L portions of hexane. The combined hexane extracts were washed three times with 1-L portions of water, dried over anhydrous magnesium sulfate and filtered under suction. The filtrate was evaporated to dryness on a rotary evaporator to give crude material (413 g, 78%) containing 90% of 3,9-di(ethylidene)-2,4,8,10-tetraoxaspiro[5,5]undecane (DETOSU) .

The crude product was dissolved in 2 L of hexane containing 10 mL of triethylamine and the solution placed in a 4-L filter flask, sealed and stored in a freezer at -20°C for two days. The crystals thus formed were collected by basket centrifugation at -5°C under an argon atmosphere. Distillation of the brownish product through a 12-inch Vigreaux column at reduced pressure gave 3,9-di-(ethylidene)-2,4,8,10-tetraoxaspiro[5,5]undecane (313 g, 61% yield) as a colorless liquid, boiling point 82°C (0.1 torr) which crystallized at room temperature, with melting point 30°C and a characteristic IR band at 1700 cm⁻¹.

### Example 2

### Preparation of Diol of Formula III(a), III(b) or III(c)

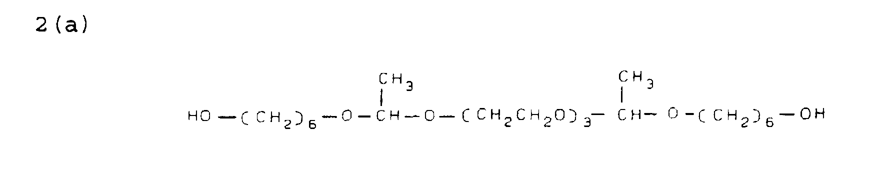

Triethyleneglycol divinyl ether (24.631 g, 100 mmoles) and 1,6-hexanediol (23.636 g, 200 mmoles) were dissolved in 100 mL of tetrahydrofuran. To this solution was added a catalytic amount (approximately 10 mg) of *p*-toluenesulfonic acid, and the mixture was refluxed under anhydrous conditions for 30 minutes. Evaporation of the solvent yielded the product shown above as a colorless oil.

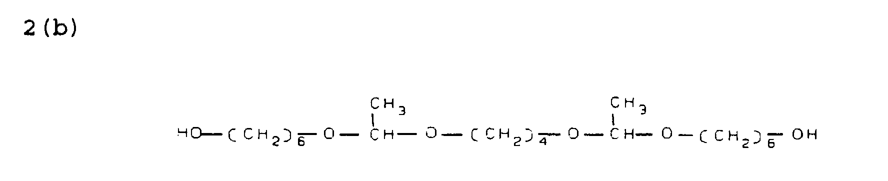

1,4-Butanediol divinyl ether (14.22 g, 100 mmoles) and 1,6-hexanediol (23.636 g, 200 mmoles) were reacted according to the procedure of Example 2(a), yielding the product shown above as an oil.

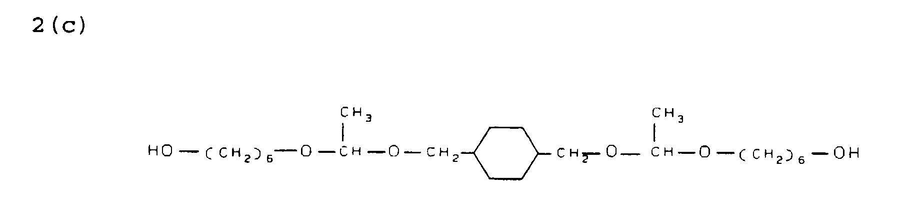

1,4-Cyclohexanedimethanol divinyl ether (19.629 g, 100 mmoles) and 1,6-hexanediol (23.636 g, 200 mmoles) were reacted according to the procedure of Example 2(a), yielding the product shown above as an oil.

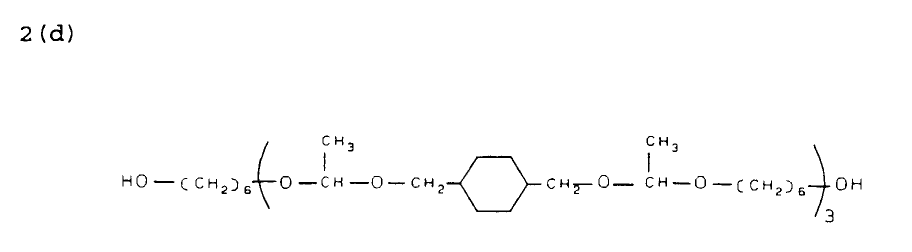

1,4-Cyclohexanedimethanol divinyl ether (5.889 g, 30 mmoles) and 1,6-hexanediol (4.727 g, 40 mmoles), were reacted according to the procedure of Example 2(a), yielding the product shown above as a viscous oil.

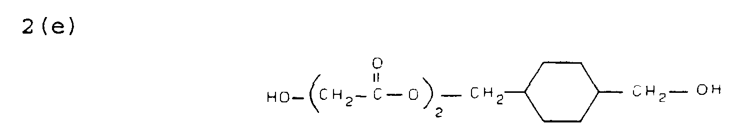

Under anhydrous conditions, 1,4-cyclohexanedimethanol (14.42 g, 100 mmoles) and glycolide (11.6 g, 100 mmoles) were weighed into a 100-mL round bottom flask. The flask was stoppered with a rubber septum, then heated in an oil bath at 180°C for 24 hours. The product shown above was obtained as a viscous oil.

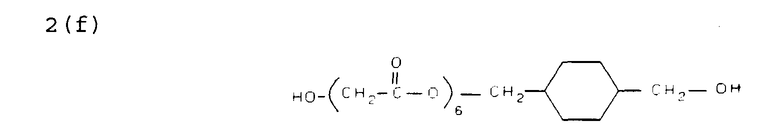

1,4-Cyclohexanedimethanol (2.88 g, 20 mmoles) and glycolide (6.96 g, 60 mmoles) were reacted according to the procedure of Example 2(e), yielding the product shown above as a low melting solid.

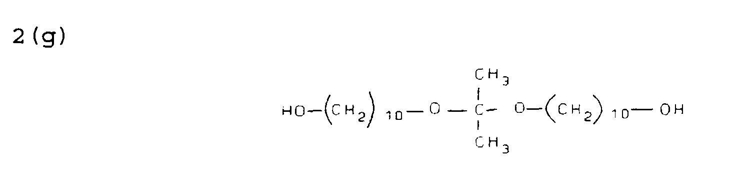

Under anhydrous conditions, 1,10-decanediol (17.43 g, 100 mmoles) and 2,2-dimethoxypropane (5.10 g, 50 mmoles) were weighed into a 250-mL flask. To the flask was added cyclohexane (150 ml) and catalytic amount of *p*-toluenesulfonic acid (20 mg). The flask was adapted to a distillation column, then heated in an oil bath at 105°C for 24 hours to remove methanol by-product by azeotropic distillation. Evaporation of the solvent yielded the product shown above as a solid material.

Using analogous procedures, the following additional diols were synthesized:

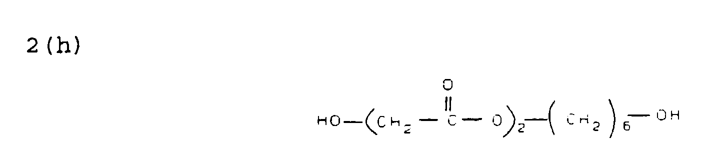

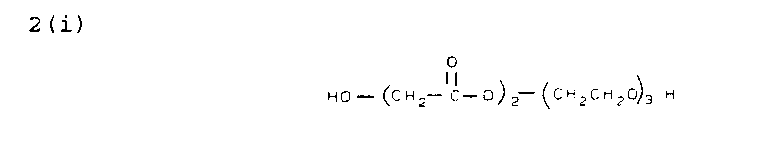

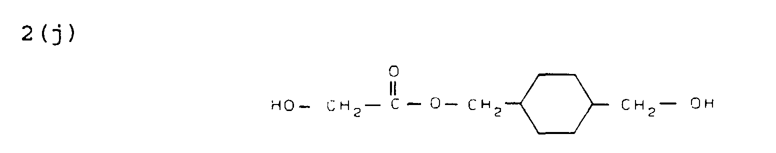

### Example 3

### Preparation of Polymer of Formula (I)

The following syntheses illustrate the preparation of polymers of this invention from the starting materials whose syntheses are shown above.

3(a) The polymer in this example was prepared from DETOSU, the diol of Example 2(a) above, *trans*-cyclohexanedimethanol (which is referred herein by the acronym *t*-CDM) and the diol of Example 2(h) above. The molar ratio of the four components (DETOSU:diol 2(a):*t*-CDM:diol 2(h)) was 100:9:90:1.

Under rigorously anhydrous conditions, DETOSU (8.49 g, 40 mmoles), diol 2(a) (1.738 g, 3.6 mmole), *t*-CDM (5.192 g, 36 mmoles) and diol 2(h) (0.094 g, 0.4 mmole) were weighed into a 250-mL round bottom flask, and the mixture dissolved in anhydrous tetrahydrofuran (75 mL). To this solution was added a *p*-toluenesulfonic acid solution in tetrahydrofuran (5 drops, 40 mg/mL) to initiate the polymerization. The solution came to a boil within a few minutes. The solution was allowed to cool to room temperature, then slowly poured with vigorous stirring into an excess of methanol (800 mL) containing a stabilizer, triethylamine (1 mL). The precipitated polymer was collected and dried overnight in a vacuum oven at 40°C. The yield was 11.8 g. The material was solid with a molecular weight of 46,500 and a glass transition temperature of 33°C.

3(b) The polymer in this example was prepared from DETOSU, the diol of Example 2(b) above, *t*-CDM and the diol of Example 2(h) above, at a molar ratio of 100:9:90:1.

Following the procedure of Example 3(a), DETOSU (8.49 g, 40 mmoles), diol 2(b) (1.363 g, 3.6 mmoles), *t*-CDM (5.192 g, 36 mmoles) and diol 2(h) (0.094 g, 0.4 mmole) were allowed to react. The reaction yielded 14.3 g of a solid material having a molecular weight of 74,000 and a glass transition temperature of 30°C.

3(c) The polymer in this example was prepared from DETOSU, the diol of Example 2(a) above, *t*-CDM and the diol of Example 2(h) above, at a molar ratio of 100:39:60:1.

Following the procedure of Example 3(a), DETOSU (8.49 g, 40 mmoles), diol 2(a) (7.541 g, 15.6 mmoles), *t*-CDM (3.46 g, 24 mmoles) and diol 2(h) (0.094 g, 0.4 mmole) were allowed to react. The reaction yielded 18.46 g of a tacky solid material having a molecular weight of 37,000.

3(d) The polymer in this example was prepared from DETOSU, the diol of Example 2(a) above, and the diol of Example 2(h) above, at a molar ratio of 100:99:1.

Following the procedure of Example 3(a), DETOSU (8.49 g, 40 mmoles), diol 2(a) (19.117 g, 39.6 mmoles) and diol 2(h) (0.094 g, 0.4 mmole) were allowed to react. The reaction yielded 18.9 g of an ointment-like material having a molecular weight of 49,000.

3(e) The polymer in this example was prepared from DETOSU and the diol of Example 2(a) above and the diol of Example 2(h) above, without including t-CDM. The molar ratio of DETOSU:diol 2(a):diol 2(h) was 100:90:10, to give a product containing no residues of *t*-CDM.

Following the procedure of Example 3(a), DETOSU (8.49 g, 40 mmoles), diol 2(a) (17.38 g, 36 mmoles) and diol 2(h) (0.94 g, 4 mmole) were allowed to react. The reaction yielded a viscous liquid material having a molecular weight of 35,000.

3(f) The polymer in this example was prepared from DETOSU, the diol of Example 2(e) above, and *t*-CDM, at a molar ratio of 100:10:90.

Following the procedure of Example 3(a), DETOSU (8.49 g, 40 mmoles), diol 2(e) (1.04 g, 4 mmoles) and *t*-CDM (5.19 g, 36 mmoles) were allowed to react. The reaction yielded 14.2 g of a solid material having a molecular weight of 84,000.

3(g) The polymer in this example was prepared from DETOSU, the diol of Example 2(e) above, and *t*-CDM, at a molar ratio of 100:50:50.

Following the procedure of Example 3(a), DETOSU (8.49 g, 40 mmoles), diol 2(e) (5.21 g, 20 mmoles) and *t*-CDM (2.884 g, 20 mmoles) were allowed to react. The reaction yielded a solid material having a molecular weight of 69,000.

3(h) The polymer in this example was prepared from DETOSU and the diol of Example 2(e) above, without including *t*-CDM. The molar ratio of DETOSU to the diol was 100:100.

Following the procedure of Example 3(a), DETOSU (8.49 g, 40 mmoles) and diol 2(e) (10.412 g, 40 mmoles) were allowed to react. The reaction yielded a solid material having a molecular weight of 105,000.

3(i) The polymer in this example was prepared from DETOSU and the diol of Example 2(f) above, without including *t*-CDM. The molar ratio of DETOSU to the diol was 100:100.

Following the procedure of Example 3(a), DETOSU (2.12 g, 10 mmoles) and diol 2(f) (4.87 g, 10 mmoles) were allowed to react. The reaction yielded a solid material having a molecular weight of 25,000.

3(j) The polymer in this example was prepared from DETOSU, the diol of Example 2(g) above, *t*-CDM and the diol of Example 2(h) above, at a molar ratio of 100:9:90:1.

Following the procedure of Example 3(a), DETOSU (8.49 g, 40 mmoles), diol 2(g) (1.391 g, 3.6 mmoles), *t*-CDM (5.192 g, 36 mmoles) and diol 2(h) (0.094 g, 0.4 mmole) were allowed to react. The reaction yielded a solid material having a molecular weight of 19,000.

Table I summarizes the products of Examples 3(a) through 3(j):

**TABLE I**

| **No.** | **Molar Ratio of Reactants** | | | | **Physical State** |
|---|---|---|---|---|---|
| 3(a) | DETOSU: 100 | 2(a) : 9 | *t*CDM: 90 | 2(h): 1 | Solid (Tg = 33°C) |
| 3(b) | DETOSU: 100 | 2(b) : 9 | *t*CDM: 90 | 2(h): 1 | Solid (Tg = 30°C) |
| 3(c) | DETOSU: 100 | 2(a) : 39 | *t*CDM: 60 | 2(h): 1 | Tacky Solid |
| 3(d) | DETOSU: 100 | 2(a) : 99 | _ | 2(h): 1 | Ointment |
| 3(e) | DETOSU: 100 | 2(a) : 90 | _ | 2(h): 10 | Ointment |
| 3(f) | DETOSU: 100 | _ | *t*CDM: 90 | 2(e): 10 | Solid |
| 3(g) | DETOSU: 100 | _ | *t*CDM: 50 | 2(e): 50 | Solid |
| 3(h) | DETOSU: 100 | _ | _ | 2(e): 100 | Solid |
| 3(i) | DETOSU: 100 | _ | _ | 2(f): 100 | Solid |
| 3(j) | DETOSU: 100 | 2(g) : 9 | *t*CDM: 90 | 2(h): 1 | Solid |

Further examples of polymers varying in rigidity are illustrated by the use of combinations of three diols (one of which was *t*-CDM), prepared in an analogous manner. The diols other than *t*-CDM are again identified by their example numbers in Example 2, and 1,6-hexanediol (designated "HD") is also included. The relative amounts of the diols in each polymer and the physical states of the polymers are shown in Table II, where two polymers containing only two diols (i.e., lacking *t*-CDM) are included for comparison. The diketene acetal in each case was again DETOSU.

**TABLE II**

| **No.** | **Diols: Molar Percentage Relative to DETOSU** | | | **Physical State** |
|---|---|---|---|---|
| 3(k) | *t*-CDM: 90 | 2(a): 9 | 2 (j ): 1 | Solid (T_{g} = 33°C) |
| 3(l) | *t*-CDM: 60 | 2(a): 39 | 2(j): 1 | Tacky Solid |
| 3(m) | | 2(a): 99 | 2(j): 1 | Ointment |
| 3(n) | *t*-CDM: 90 | 2(b): 9 | 2(i): 1 | Solid (T_{g} = 30°C) |
| 3(o) | *t*-CDM: 60 | 2(b): 39 | 2(i): 1 | Tacky Solid |
| 3(p) | | 2(b): 99 | 2(i): 1 | Ointment |
| 3(q) | *t*-CDM: 90 | 2(c): 9 | 2 (h): 1 | Solid (T_{g} = 58°C) |
| 3(r) | *t*-CDM: 50 | HD: 30 | 2(e): 20 | Tacky, Soft Solid |
| 3(s) | *t*-CDM: 50 | HD: 30 | 2(e): 20 | Tacky, Soft Solid |

Cross-linked polymers may also be prepared as demonstrated in the following examples:

3(t) DETOSU (2.12 g, 10 mmole) and diol 2(i) (1.33 g, 5 mmole) were allowed to react in tetrahydrofuran. After the reaction was completed, tetrahydrofuran was removed to yield a viscous liquid which was then mixed with 1,2,6-hexanetriol (0.456 g, 3.4 mmole). The resulting liquid mixture was heated at 70°C under anhydrous conditions for 10 hours. The reaction yielded a colorless flexible solid material.

3(u) DETOSU (2.12 g, 10 mmole), 1,6-hexanediol (0.473 g, 4 mmole), diol 2(i) (0.266 g, 1 mmole) and 1,2,6-hexanetriol (0.456 g, 3.4 mmole) were thoroughly mixed. The material was cured following the procedure of Example 3(t) which yielded a flexible solid material.

### Example 4

### Preparation of Bioerodible Device & Drug Release Study

The following syntheses illustrate further preparations of polymers of this invention, and how the rate of bioerodibility can be controlled by varying the amount of hydrolyzable ester present in the polymer.

(a) A series of polymers were prepared by reacting DETOSU with the diol of Example 2(e) and *t*-CDM, varying the proportion of the 2(e) diol to *t*-CDM while maintaining the total moles of both equal to the total moles of DETOSU. The molar ratios of the α-hydroxy acid containing diol 2(e) to *t*-CDM used were 0:100, 25:75, 50:50, 75:25, and 90:10.

The rates of bioerodibility of these polymers were then determined by weight loss tests conducted by pressing each polymer into a film 0.7 mm thick on a Carver press. Discs measuring 7 mm in diameter were then cut from the films. Each disc was placed in 10 mL of pH 7.4 phosphate buffer and incubated at 37°C. Periodically, a disc was removed and dried, and its weight loss was determined gravimetrically.

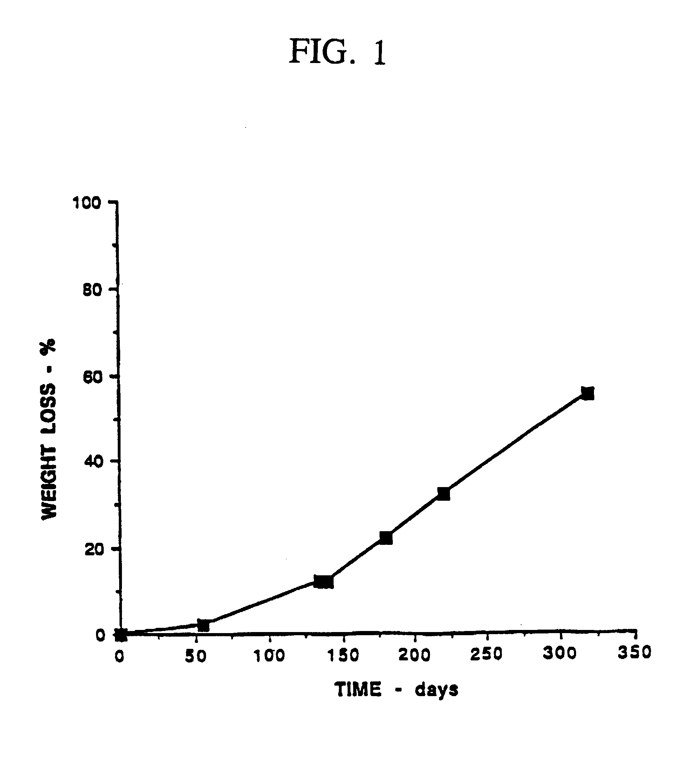

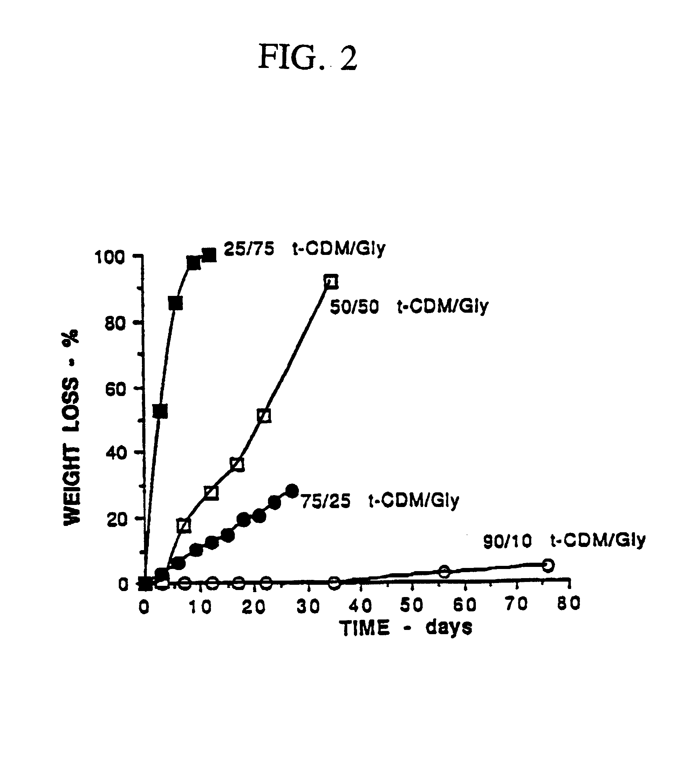

The results are plotted in FIGS. 1 and 2. FIG. 1 shows the weight loss of a polymer that does not contain the glycolide moiety, as a function of time over a period of 350 days. FIG. 2 shows the weight loss over 80 days of the four glycolide-containing polymers, with the filled squares representing the polymer with the 25:75 molar ratio, the open squares the polymer with the 50:50 molar ratio, the filled circles the polymer with the 75:25 molar ratio, and the open circles the polymer with the 90:10 molar ratio. The comparison in FIG. 2 and between FIGS. 1 and 2 demonstrates that an increase in the proportion of the hydrolyzable glycolic acid ester moiety present in the polymer results in an increase in bioerodibility.

(b) An ester-containing diol was prepared by reacting equimolar amounts of triethylene glycol (TEG) and glycolide (GLY) by procedures analogous to those described above. The resulting diol (TEG/GLY) was then used to prepare a series of polymers by reacting with DETOSU and *t*-CDM, using varying proportions of the (TEG/GLY) diol to *t*-CDM. A polymer having a (TEG/GLY) diol to *t*-CDM molar ratio of 10:90 was compared with a polymer having a (TEG/GLY) diol to *t*-CDM molar ratio of 20:80 and one having a (TEG/GLY) diol to *t*-CDM molar ratio of 25:75 in a test measuring the sustained release of 5-fluorouracil. This test was conducted by dissolving the polymers in tetrahydrofuran and adding 5-fluorouracil powder at 10% by weight to form suspensions. The suspensions were then stirred under a nitrogen flow to remove solvent, and residual solvent was removed by placing the mixtures in a vacuum oven. The mixtures were then pressed into films and cut into discs. Each disc was placed in 25 mL of pH 7.4 phosphate buffer at 37°C, and the amounts of 5-fluorouracil released were determined by measuring the UV absorption of the buffer solutions at 265 nm.

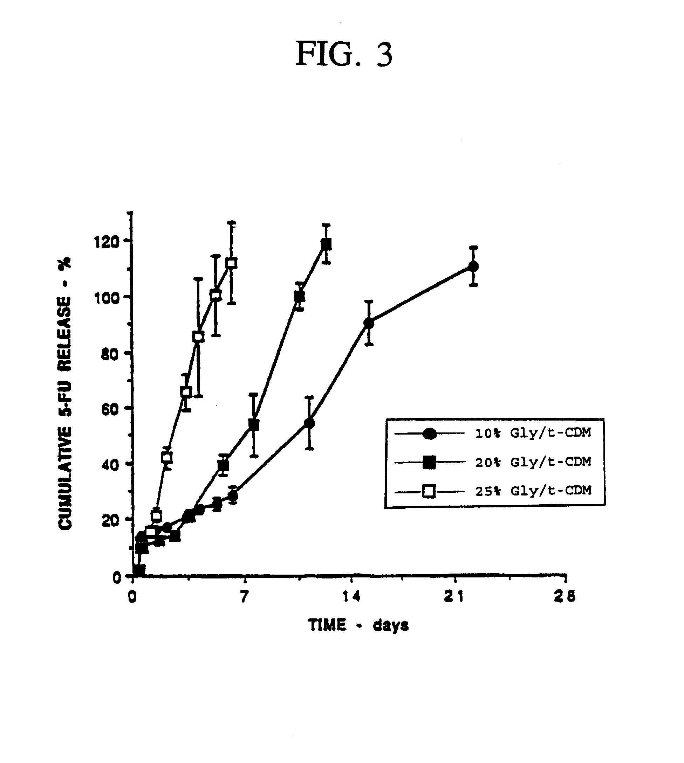

The results are plotted in FIG. 3, where the filled circles represent the 10:90 ratio, the filled squares represent the 20:80 ratio, and the open squares the 25:75 ratio. The plot demonstrates that an increase in the proportion of hydrolyzable ester moiety present in the polymer results in an increase in bioerodibility.

Further examples of polymers varying in their rate of bioerodibility are listed in Table III. In the first three rows of the table, the diols are triethylene glycol (TEG) and triethylene glycol glycolide (identified by reference to its synthesis in Example 2(i)), and the degree of erodibility increases as the proportion of TEG glycolide increases. In the last three rows of the table the diols are *t*-CDM, 1,6-hexanediol (HD) and 1,6-hexanediol glycolide (2(h)), and the degree of erodibility again increases as the proportion of the glycolide increases. The diketene acetal was again DETOSU.

**TABLE III**

| **No.** | **Diols: Molar Percentage Relative to DETOSU** |
|---|---|
| 4(a) | TEG: 0 2(i): 100 |
| 4(b) | TEG: 50 2(i): 50 |
| 4(c) | TEG: 90 2(i): 10 |
| 4(d) | *t*-CDM: 60 2(h): 40 HD: 0 |
| 4(e) | *t*-CDM: 60 2(h): 20 HD: 20 |
| 4(f) | *t*-CDM: 60 2(h): 5 HD: 35 |

The foregoing is offered primarily for purposes of illustration. It will be readily apparent to those skilled in the art that the molecular structures, proportions of the reactant materials, methods of use and other parameters of the invention described herein may be further modified or substituted in various ways without departing from the spirit and scope of the invention.

## Claims

1. A polymer of Formula I 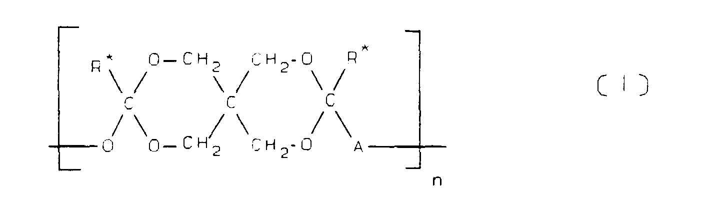 wherein R* is a C₁-C₄ alkyl, preferably methyl or ethyl, A is -O-R¹-, -O-R²- or (-O-R³)_{q}-, n is ≥5; and
R¹ is 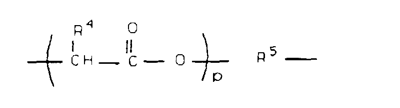 in which:
p is 1-10;
R⁴ is hydrogen or a C₁-C₆ alkyl; and
R⁵ is 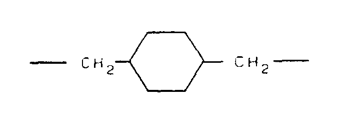 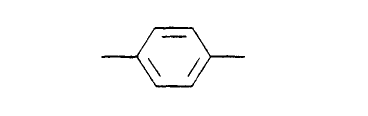 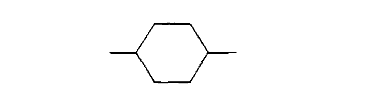 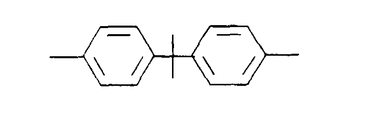 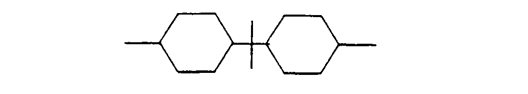 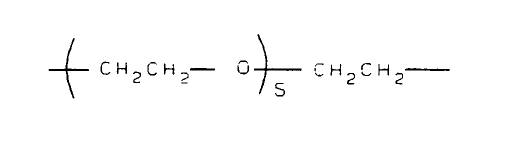
(̵CH₂)̵ₜ
or 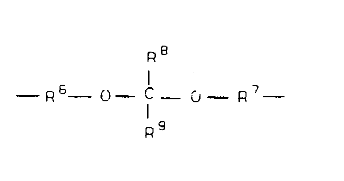
wherein:
s is 1 to 100;
t is 1 to 12;
R⁶ and R⁷ are independently a C₁-C₁₂ alkylene;
R⁸ is hydrogen or a C₁-C₆ alkyl; and
R⁹ is a C₁-C₆ alkyl; or
R⁸ and R⁹ taken together are a C₃-C₁₀ alkylene;
R² is 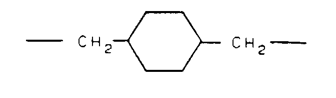 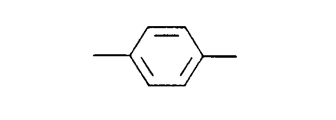 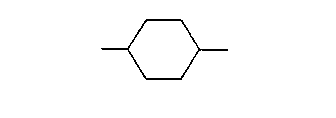 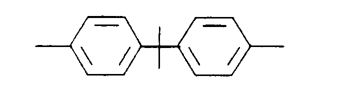 or 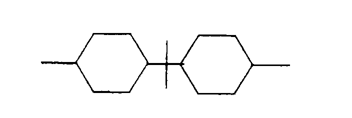
and in (-O-R³)_{q}-, q is 1 to 20;
when q is 1, R³ is 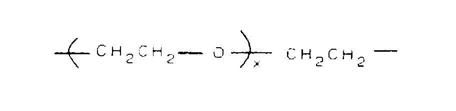 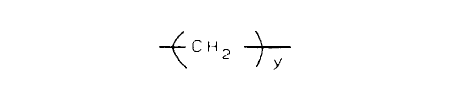 or 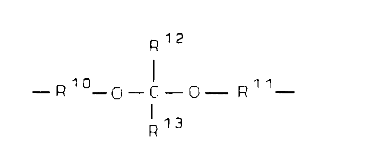 in which:
x is 1 to 100;
y is 1 to 12;
R¹⁰ and R¹¹ are independently a C₁-C₁₂ alkylene;
R¹² is hydrogen or a C₁-C₆ alkyl; and
R¹³ is a C₁-C₆ alkyl; or
R¹² and R¹³ taken together are a C₃-C₁₀ alkylene; and
when q is 2 to 20, each R³ may be the same or different and is selected from the group consisting of 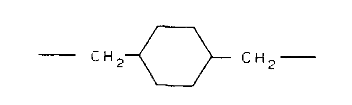 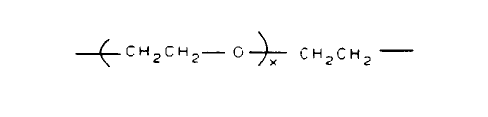 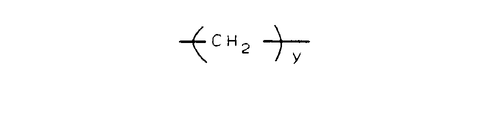 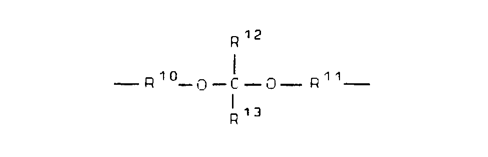 and 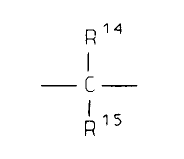
wherein x, y, R¹⁰, R¹¹, R¹² and R¹³ are as defined above, R¹⁴ is hydrogen or a C₁-C₄ alkyl, and R¹⁵ is a C₁-C₄ alkyl, with the proviso that the polymer has at least 0.1 mole percent of the unit in which A is -O-R¹-.

2. The polymer of Claim 1 wherein n is about 5 to about 1000, preferably about 20 to about 500, more preferably about 30 to about 300.

3. The polymer of Claim 1 comprising about 1 to about 50 mole percent of the unit in which A is -O-R¹-.

4. The polymer of Claim 3 comprising about 2 to about 30 mole percent of the unit in which A is -O-R¹-.

5. The polymer of Claim 1 wherein the unit in which A is -O-R²- constitutes up to about 20 mole percent of the polymer.

6. The polymer of Claim 1 wherein the unit in which A is -O-R²- constitutes about 60 to about 99.9 mole percent of the polymer.

7. The polymer of Claim 1 comprising the unit wherein A is -O-R¹- and R¹ is 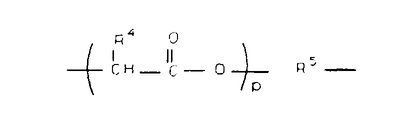 in which R⁴ and R⁵ are as defined in Claim 1 and p is 1 to 6, preferably 1 to 4 and more preferably 1 or 2.

8. The polymer of Claim 7 wherein R⁴ is hydrogen or methyl.

9. The polymer of Claim 8 wherein R⁴ is hydrogen or methyl and R⁵ is selected from a group consisting of 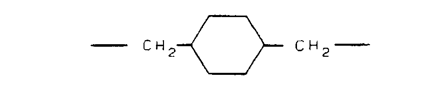 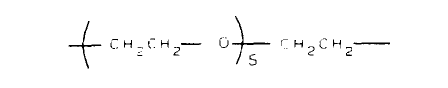 wherein s is 2 to 6, preferably 2;
and
(̵CH₂)̵ₜ
wherein t is 4 to 6, preferably 6.

10. The polymer of Claim 9 comprising the unit wherein A is -O-R¹- and R¹ is 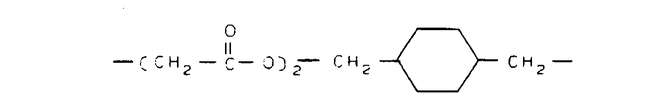 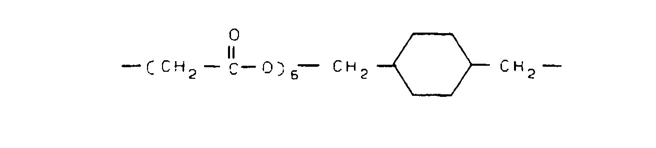 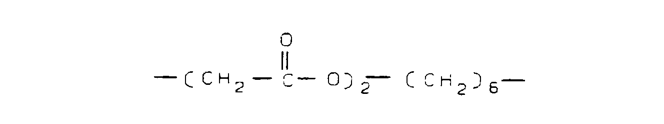 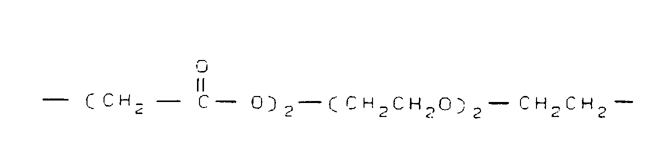 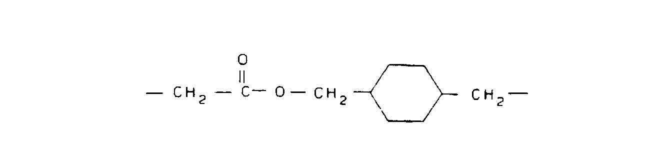 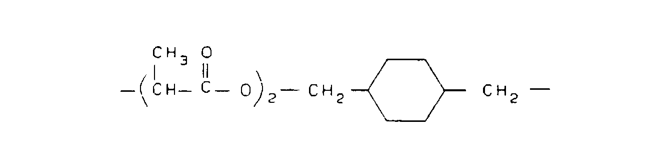 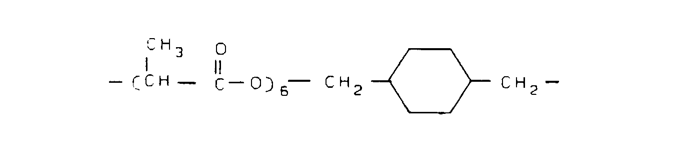 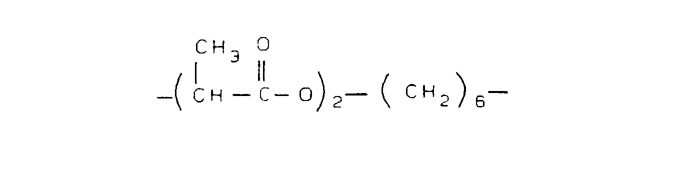 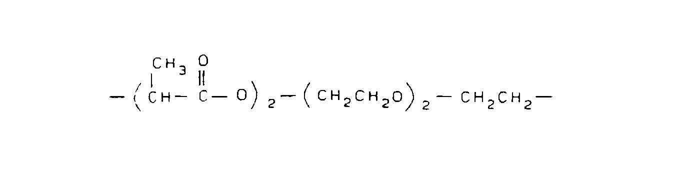 or

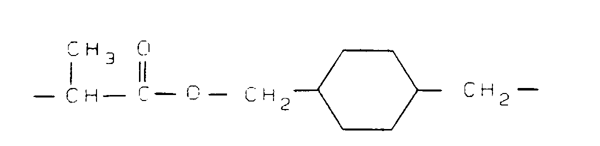

11. The polymer of Claim 8 wherein R⁵ is 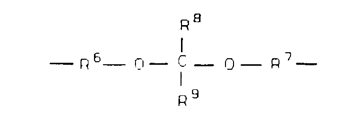 in which R⁶ and R⁷ are identical and are an unbranched C₄-C₁₂ alkylene, preferably an unbranched C₆-C₁₂ alkylene and R⁸ and R⁹ are as defined in Claim 1.

12. The polymer of Claim 11 wherein R⁸ is hydrogen and R⁹ is methyl.

13. The polymer of Claim 1 comprising the unit wherein A is -O-R²- and R² is

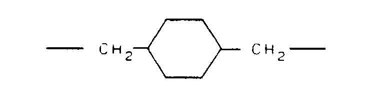

14. The polymer of Claim 1 comprising the unit wherein A is (-O-R³)_{q}- in which q is 1 to 6 preferably 1 to 3, and R³ is as defined in Claim 1.

15. The polymer of Claim 14 wherein q is 2 to 6 and each R³ is selected from a group consisting of 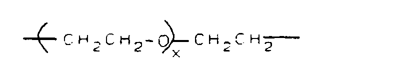 wherein x is 2 to 6, preferably 2, 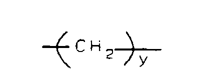 wherein y is 4 to 6, preferably 6, 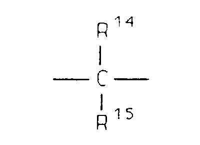 wherein R¹⁴ is hydrogen and R¹⁵ is methyl
and

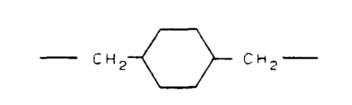

16. The polymer of Claim 15 comprising the unit wherein A is (-O-R³)_{q}- and (-O-R³)_{q}- is 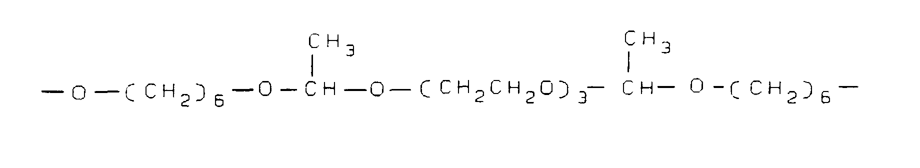 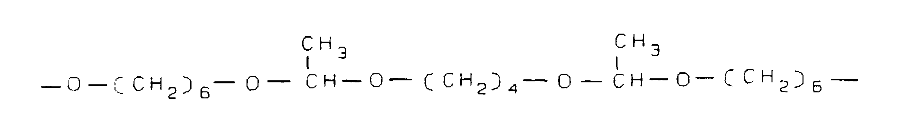 or

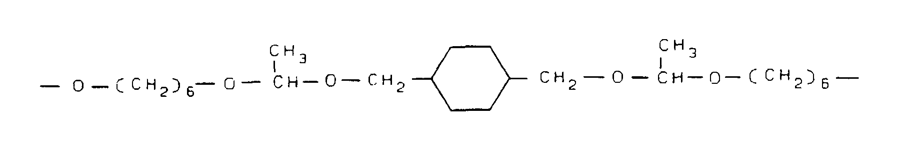

17. The polymer of Claim 14 wherein q is 1 and R³ is 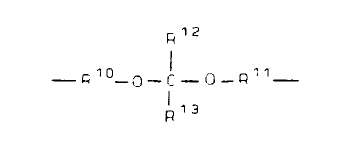 in which both R¹⁰ and R¹¹ are an unbranched C₄-C₁₂ alkylene, preferably an unbranched C₆-C₁₂ alkylene and R¹² and R¹³ are both methyl.

18. The polymer of Claim 17 wherein R¹⁰ and R¹¹ are both an unbranched C₁₀ alkylene.

19. The polymer of Claims 7-12 further optionally comprising either the unit wherein A is -O-R²- and R² is 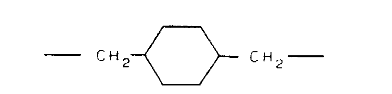 or the unit wherein A is (-O-R³)_{q}- and R³ and q are as defined in any of Claims 14-18, or both.

20. A composition comprising a matrix composed of a polymer of Claim 1 and an active agent.

21. The use of a polymer of Claim 1 for the preparation of a composition for sustained release of an active agent.

22. The composition of Claim 20 or the use of Claim 21 wherein said active agent is a pharmaceutical, cosmetic or agricultural agent, preferably a pharmaceutical agent such as an antigen, a vaccine, a polypeptide, a hormonal agent, an antipsychotic agent or an anti-neoplastic agent.

23. A polymer of Claim 1 for use in orthopedic restoration or tissue regeneration.

24. A process for the preparation of a polymer comprising individual units of the following formula 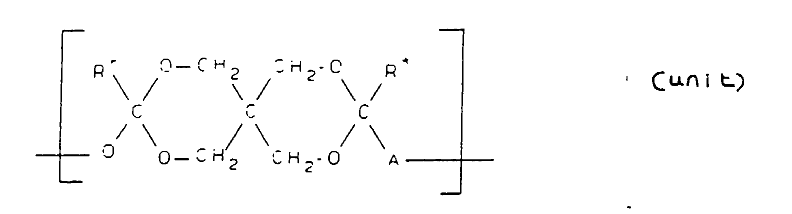 wherein R* is a C₁-C₄ alkyl preferably methyl or ethyl, A is -O-R¹-, -O-R²- or (-O-R³)_{q}-; and
R¹ is 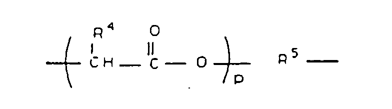 in which:
p is 1-10;
R⁴ is hydrogen or a C₁-C₆ alkyl; and
R⁵ is 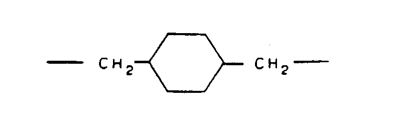 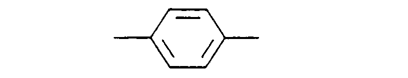 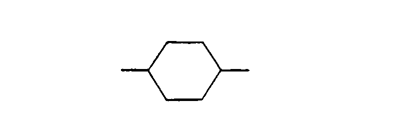 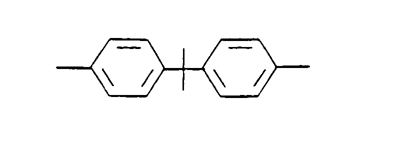 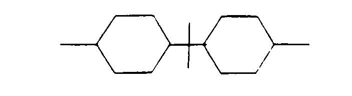 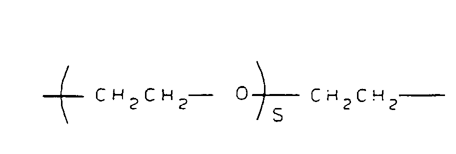
(̵CH₂)̵ₜ
or 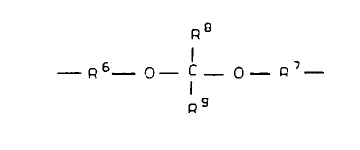
wherein:
s is 1 to 100;
t is 1 to 12;
R⁶ and R⁷ are independently a C₁-C₁₂ alkylene;
R⁸ is hydrogen or a C₁-C₆ alkyl; and
R⁹ is a C₁-C₆ alkyl; or
R⁸ and R⁹ taken together are a C₃-C₁₀ alkylene;
R² is 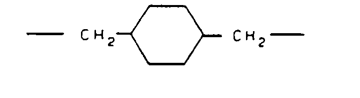 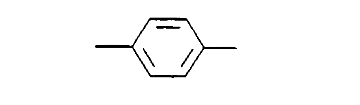 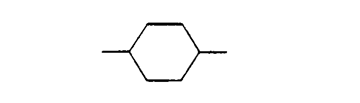 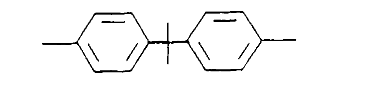 or 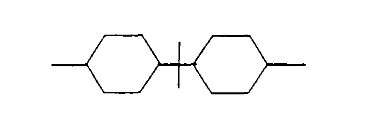
and in (-O-R³)_{q}-, q is 1 to 20; when q is 1, R³ is 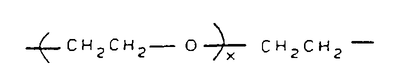 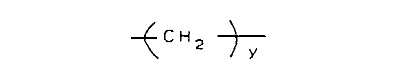 or 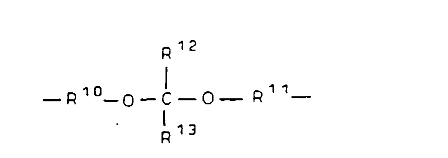 in which:
x is 1 to 100;
t is 1 to 12;
R¹⁰ and R¹¹ are independently a C₁-C₁₂ alkylene;
R¹² is hydrogen or a C₁-C₆ alkyl; and
R¹³ is a C₁-C₆ alkyl; or
R¹² and R¹³ taken together are a C₃-C₁₀ alkylene; and
when q is 2 to 20, each R³ may be the same or different and is selected from the group consisting of 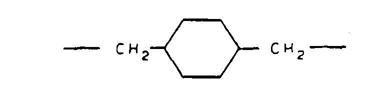 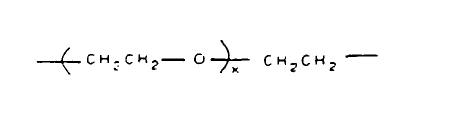 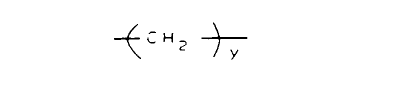 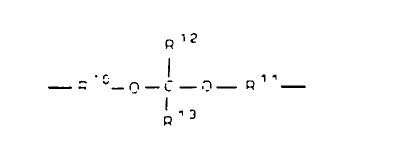 and 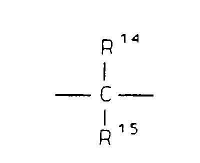
wherein x, y, R¹⁰, R¹¹, R¹² and R¹³ are as defined above, R¹⁴ is hydrogen or a C₁-C₄ alkyl and R¹⁵ is a C₁-C₄ alkyl, with the proviso that the polymer has at least 0.1 mole percent of the unit in which A is -O-R¹-, which process comprises reacting a diketene acetal of the formula 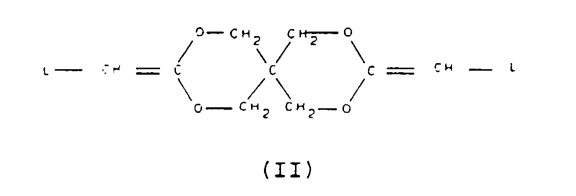 in which L is hydrogen or a C₁₋₃ alkyl; with a diol of the following Formula III(a) or a mixture of two or three of the diols of the following Formulas III(a), III(b) or III(c) in selected proportions:
HO-R¹-OH III(a)
HO-R²-OH III(b)
H(-O-R³)_{q}-OH III(c)
wherein R¹, R², R³ and q are as defined above in the preamble, with the proviso that at least 0.1 mole percent in the diol mixture is a diol of Formula III(a).

25. The process of Claim 24 wherein a said mixture of diols is used and comprises 1 to 50 mole percent of the diol of Formula III(a).

26. The process of Claim 25 wherein a said mixture of diols is used and comprises 2 to 30 mole percent of the diol of Formula III(a).

27. The process of Claim 24 wherein a said mixture of diols is used and comprises up to 20 mole percent of the diol of Formula III(b).

28. The process of Claim 24 wherein a said mixture of diols is used and comprises 60 to 99.9 mole percent of the diol of Formula III(b).

29. The process of Claim 24 wherein a said mixture of diols is used and comprises a diol of Formula III(a) wherein R¹ is 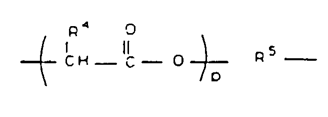 in which R⁴ and R⁵ are as defined in Claim 24 and p is 1 to 6.

30. The process of claim 29 wherein P is 1 to 4 and preferably 1 or 2.

31. The process of claim 29 or claim 30 wherein R⁴ is hydrogen or methyl.

32. The process of claim 31 wherein R⁴ is hydrogen or methyl and R⁵ is 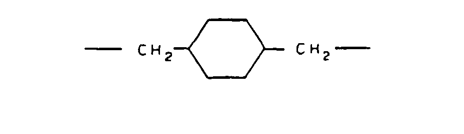 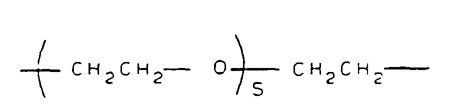 wherein s is 2 to 6,
or
(̵CH₂)̵ₜ
wherein t is 4 to 6.

33. The process of claim 32 wherein s is 2 and t is 6.

34. The process of claim 32 or claim 33 wherein R¹ in the diol of Formula III(a) is 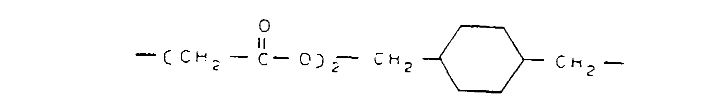 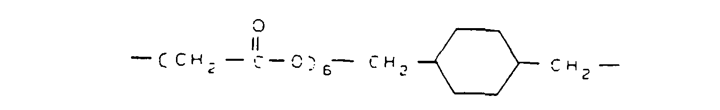 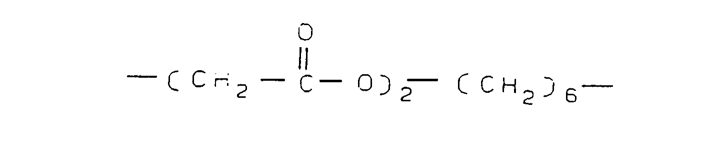 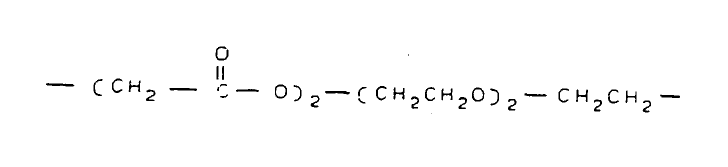 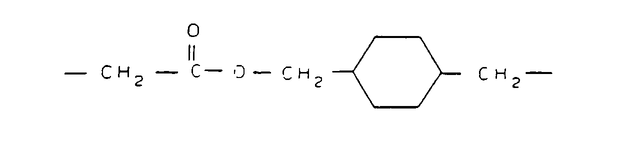 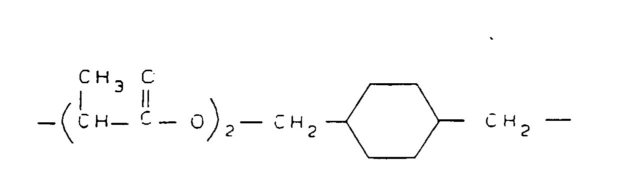 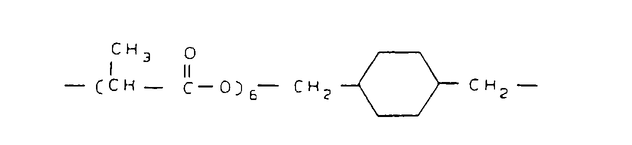 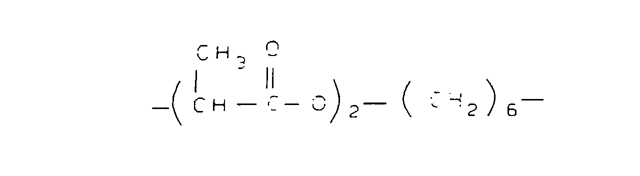 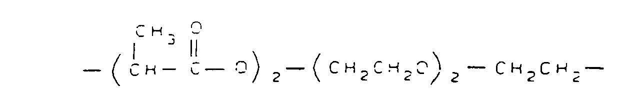 or

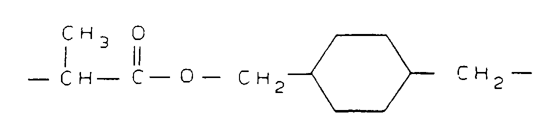

35. The process of Claim 31 wherein R⁵ is 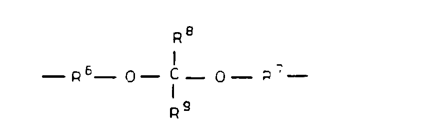 in which R⁶ and R⁷ are identical and are an unbranched C₄-C₁₂ alkylene, and R⁸ and R⁹ are as defined in Claim 24.

36. The process of claim 35 wherein R⁶ and R⁷ are an unbranched C₆-C₁₂ alkylene.

37. The process of Claim 35 or 36 wherein R⁸ is hydrogen and R⁹ is methyl.

38. The process of Claim 24 wherein a said mixture of diols is used and comprises a diol of Formula III(b) in which R² is

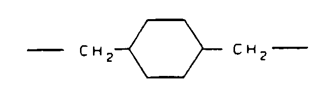

39. The process of Claim 24 wherein said mixture of diols comprising a diol of Formula III(c) in which q is 1 to 6 and R³ is as defined in Claim 24.

40. The process of claim 39 wherein q is 1 to 3.

41. The process of Claim 39 wherein q is 2 to 6 and each R³ is selected from a group consisting of 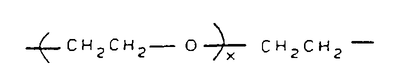 wherein x is 2 to 6, preferably 2; 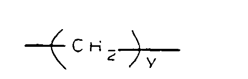 wherein y is 4 to 6, preferably 6; 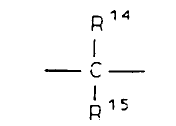 wherein R¹⁴ is hydrogen and R¹⁵ is methyl
and

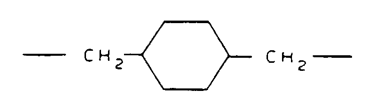

42. The process of Claim 41 wherein (-O-R³)_{q}- is 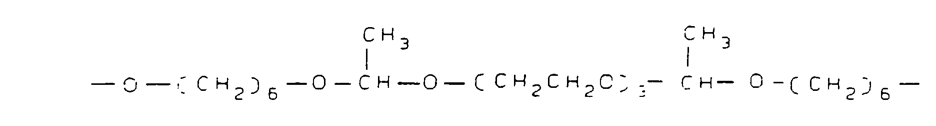 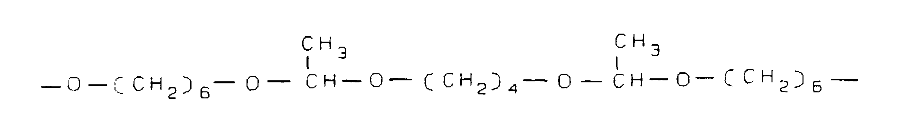 or

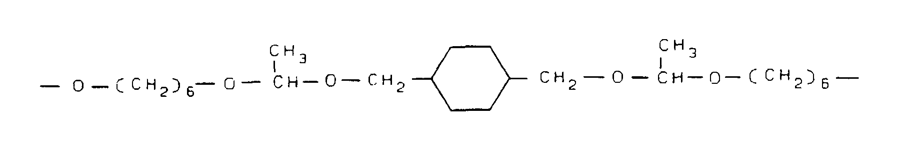

43. The process of Claim 39 wherein q is 1 and R³ is 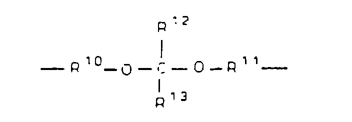 in which both R¹⁰ and R¹¹ are an unbranched C₄-C₁₂ alkylene, and R¹² and R¹³ are both methyl.

44. The process of claim 43 wherein R¹⁰ and R¹¹ are an unbranched C₆-C₁₂ alkylene.

45. The process of claim 44 wherein R¹⁰ and R¹¹ are both an unbranched C₁₀ alkylene.

46. The process of any one of claims 24 to 45 which includes cross-linking with a polyol containing more than two hydroxy groups.

47. A polymer obtainable by reaction between a diketene acetal of Formula (II) with a mixture of diols comprising at least 0.1 mole percent of a diol of Formula III(a) in which R¹ is as defined in any of Claims 29-37 and further comprising either a diol of Formula III(b) in which R² is 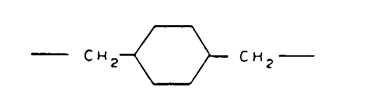 or a diol of Formula III(c) in which R³ and q are as defined in any of Claims 39-45, or both.

48. A polymer obtainable by reaction between:
(a) a diketene acetal of the formula 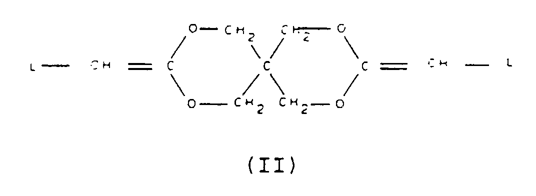 in which L is hydrogen or a C₁₋₃ alkyl; and
(b) at least one diol and at least one polyol with more than two hydroxy groups at least 0.1 mole percent of which is an α-hydroxy-acid-containing diol of Formula III(a) as defined in any of the preceding claims.

## Patentansprüche

1. Polymer der Formel (I) 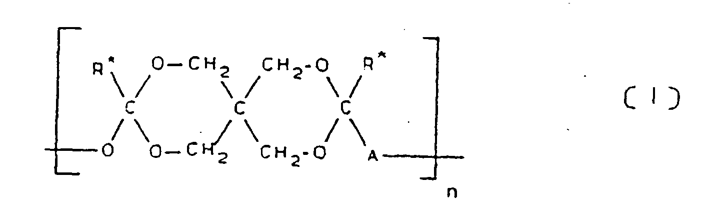 worin R* ein C₁-C₄-Alkyl, bevorzugt Methyl oder Ethyl, ist, A -O-R¹-, -O-R²- oder (-O-R³)_{q}- ist, n ≥ 5 ist; und R¹ 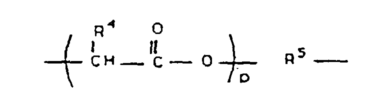 ist, worin:
p 1 bis 10 ist;
R⁴ Wasserstoff oder ein C₁-C₆-Alkyl ist; und
R⁵ 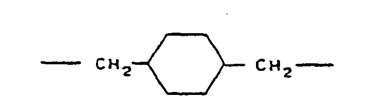 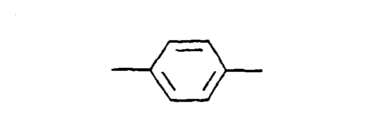 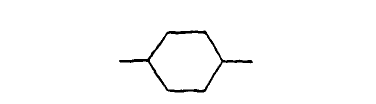 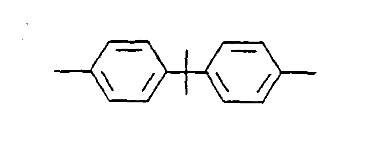 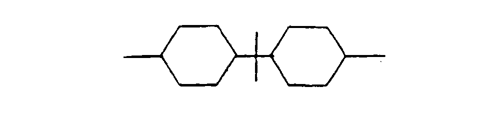 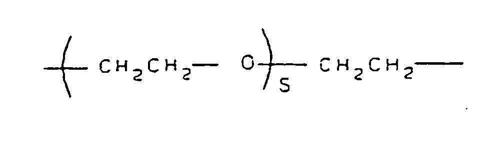
(̵CH₂)̵ₜ
oder 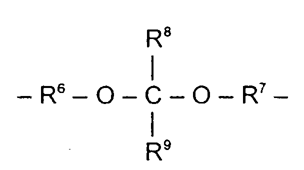 ist, worin:
s 1 bis 100 ist;
t 1 bis 12 ist;
R⁶ und R⁷ unabhängig ein C₁-C₁₂-Alkylen sind;
R⁸ Wasserstoff oder ein C₁-C₆-Alkyl ist; und
R⁹ ein C₁-C₆-Alkyl ist; oder
R⁸ und R⁹ zusammengenommen ein C₃-C₁₀-Alkylen sind;
R² 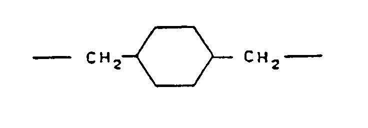 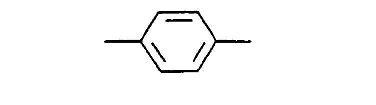 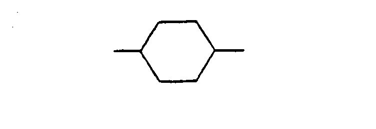 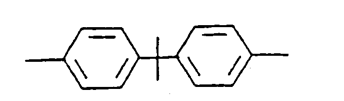 oder 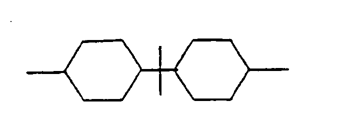
ist und in (-O-R³)_{q}- q 1 bis 20 ist;
und wenn q 1 ist, R³ 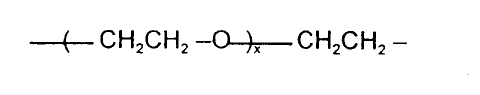 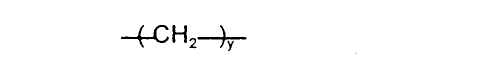 oder 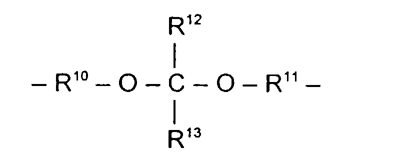 ist, worin:
x 1 bis 100 ist;
y 1 bis 12 ist;
R¹⁰ und R¹¹ unabhängig ein C₁-C₁₂-Alkylen sind;
R¹² Wasserstoff oder ein C₁-C₆-Alkyl ist; und
R¹³ ein C₁-C₆-Alkyl ist; oder
R¹² und R¹³ zusammengenommen ein C₃-C₁₀-Alkylen sind; und
wenn q 2 bis 20 ist, jedes R³ gleich oder verschieden sein kann und ausgewählt ist aus der Gruppe bestehend aus 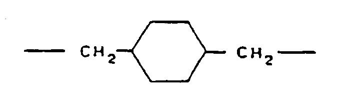 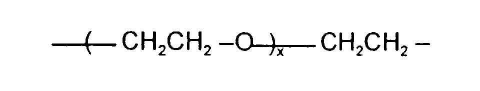 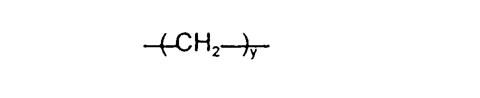 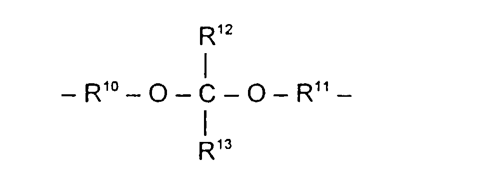 und 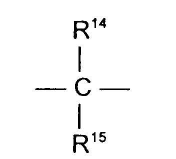
worin x, y, R¹⁰, R¹¹, R¹² und R¹³ wie oben definiert sind, R¹⁴ Wasserstoff oder ein C₁-C₄-Alkyl ist und R¹⁵ ein C₁-C₄-Alkyl ist, mit der Maßgabe, daß das Polymer mindestens 0,1 Mol-% der Einheit, in der A -O-R¹- ist, aufweist.

2. Polymer nach Anspruch 1, worin n etwa 5 bis 1000, bevorzugt etwa 20 bis 500, noch mehr bevorzugt etwa 30 bis 300, ist.

3. Polymer nach Anspruch 1, umfassend etwa 1 bis 50 Mol-% der Einheit, in der A-O-R¹- ist.

4. Polymer nach Anspruch 3, umfassend etwa 2 bis 30 Mol-% der Einheit, in der A-O-R¹- ist.

5. Polymer nach Anspruch 1, worin die Einheit, in der A -O-R²- ist, bis zu etwa 20 Mol-% des Polymers bildet.

6. Polymer nach Anspruch 1, worin die Einheit, in der A -O-R²- ist, etwa 60 bis 99,9 Mol-% des Polymers bildet.

7. Polymer nach Anspruch 1, umfassend die Einheit, worin A -O-R¹- ist und R¹ 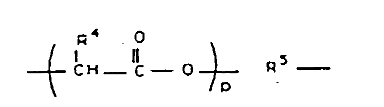 ist, worin R⁴ und R⁵ wie in Anspruch 1 definiert sind und p 1 bis 6, bevorzugt 1 bis 4 und noch mehr bevorzugt 1 oder 2, ist.

8. Polymer nach Anspruch 7, worin R⁴ Wasserstoff oder Methyl ist.

9. Polymer nach Anspruch 8, worin R⁴ Wasserstoff oder Methyl ist und R⁵ ausgewählt ist aus der Gruppe bestehend aus 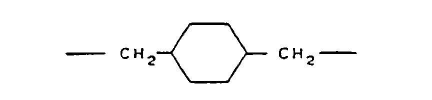 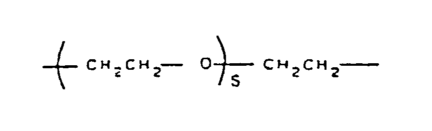 worin s 2 bis 6, bevorzugt 2, ist;
und 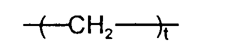 worin t 4 bis 6, bevorzugt 6, ist.

10. Polymer nach Anspruch 9, umfassend die Einheit, in der A -O-R¹- ist und R¹ 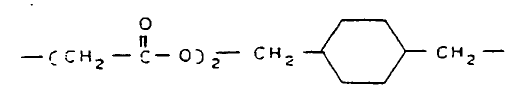 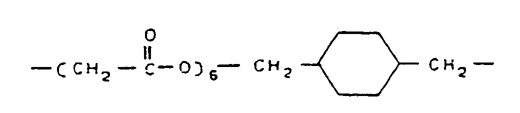 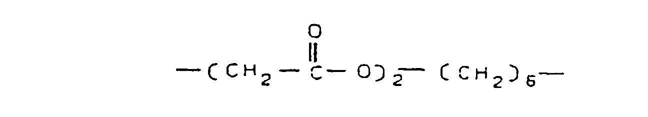 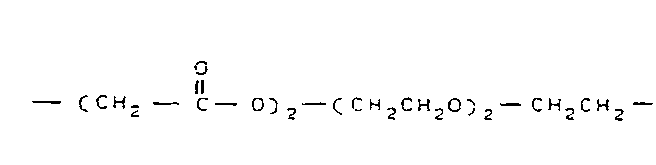 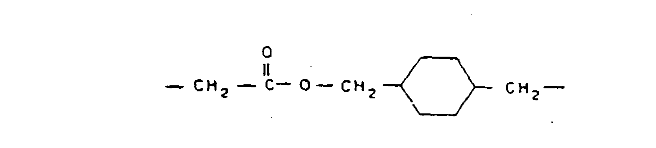 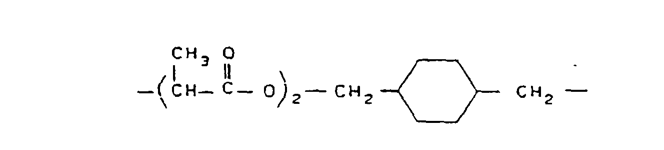 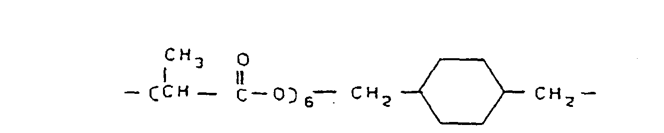 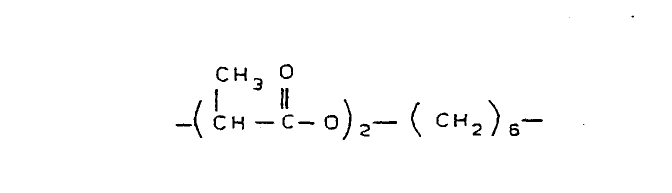 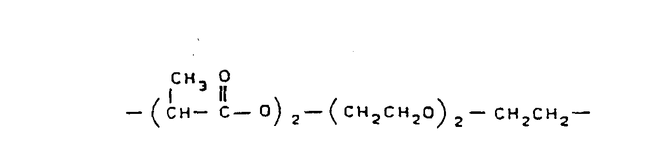 oder 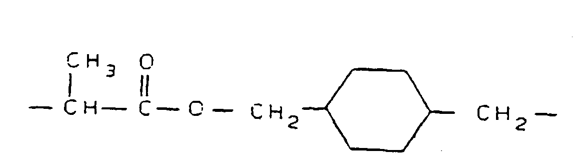 ist.

11. Polymer nach Anspruch 8, worin R⁵ 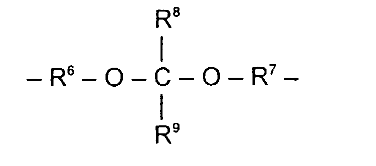 ist, worin R⁶ und R⁷ identisch sind und ein unverzweigtes C₄-C₁₂-Alkylen, bevorzugt ein unverzweigtes C₆-C₁₂-Alkylen, darstellen und R⁸ und R⁹ wie in Anspruch 1 definiert sind.

12. Polymer nach Anspruch 11, worin R⁸ Wasserstoff und R⁹ Methyl ist.

13. Polymer nach Anspruch 1, umfassend die Einheit, in der A -O-R²- ist und R² 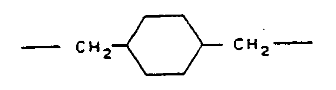 ist.

14. Polymer nach Anspruch 1, umfassend die Einheit, in der A (-O-R³)_{q}- ist, worin q 1 bis 6, bevorzugt 1 bis 3, ist und R³ wie in Anspruch 1 definiert ist.

15. Polymer nach Anspruch 14, worin q 2 bis 6 ist und jedes R³ ausgewählt ist aus der Gruppe bestehend aus 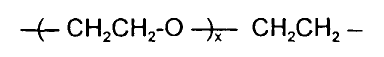 worin x 2 bis 6, bevorzugt 2, ist, 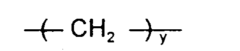 worin y 4 bis 6, bevorzugt 6, ist, 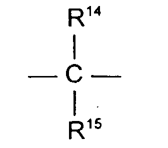 worin R¹⁴ Wasserstoff und R¹⁵ Methyl ist
und

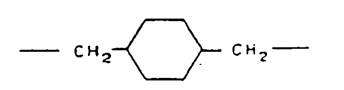

16. Polymer nach Anspruch 15, umfassend die Einheit, in der A (-O-R³)_{q}- ist und (-O-R³)_{q}- 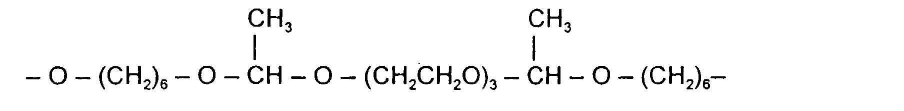 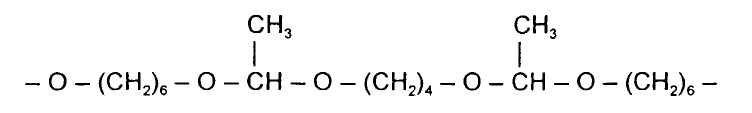 oder 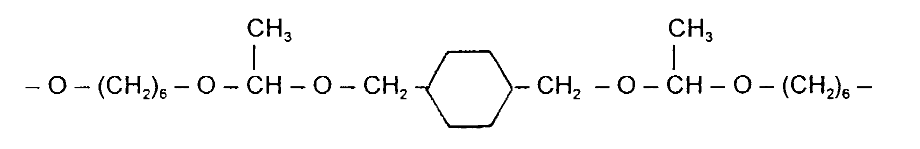 ist.

17. Polymer nach Anspruch 14, worin q 1 ist und R³ 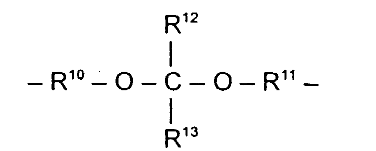 ist, worin R¹⁰ und R¹¹ beide ein unverzweigtes C₄-C₁₂-Alkylen, bevorzugt ein unverzweigtes C₆-C₁₂-Alkylen, sind und R¹² und R¹³ beide Methyl sind.

18. Polymer nach Anspruch 17, worin R¹⁰ und R¹¹ beide ein unverzweigtes C₁₀-Alkylen sind.

19. Polymer nach den Ansprüchen 7 bis 12, das ferner wahlweise entweder die Einheit, in der A -O-R²- ist und R² 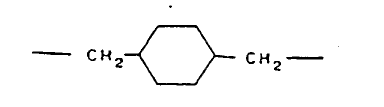 ist, oder die Einheit, in der A (-O-R³)_{q}- ist und R³ und q wie in irgendeinem der Ansprüche 14 bis 18 definiert sind, oder beide umfaßt.

20. Zusammensetzung, umfassend eine Matrix aus einem Polymer nach Anspruch 1 und einen Wirkstoff.

21. Verwendung eines Polymers nach Anspruch 1 zur Herstellung einer Zusammensetzung für die verzögerte Freigabe eines Wirkstoffs.

22. Zusammensetzung nach Anspruch 20 oder Verwendung nach Anspruch 21, worin der Wirkstoff ein pharmazeutisches, kosmetisches oder ein die landwirtschaftliches Mittel, bevorzugt ein pharmazeutisches Mittel, wie ein Antigen, ein Impfstoff, ein Polypeptid, ein hormonelles Mittel, ein Antipsychotikum oder ein antineoplastisches Mittel, ist.

23. Polymer nach Anspruch 1 zur Verwendung bei der orthopädischen Wiederherstellung oder bei der Geweberegeneration.

24. Verfahren zur Herstellung eines Polymers, umfassend einzelne Einheiten der folgenden Formel 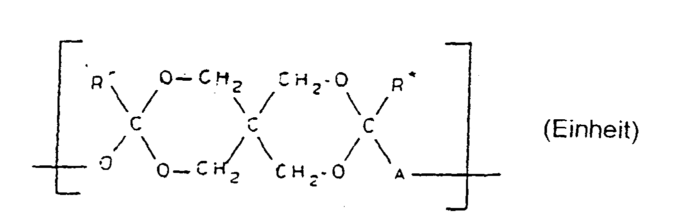 worin R* ein C₁-C₄-Alkyl, bevorzugt Methyl oder Ethyl, ist, A -O-R¹-, -O-R²- oder (-O-R³)_{q}- ist; und R¹ 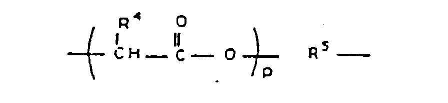 ist, worin:
p 1 bis 10 ist;
R⁴ Wasserstoff oder ein C₁-C₆-Alkyl ist; und
R⁵ 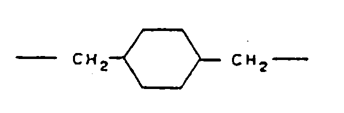 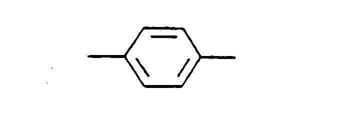 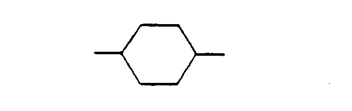 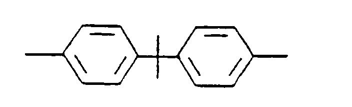 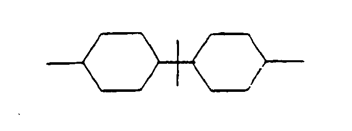 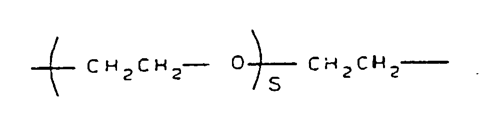
(̵CH₂)̵ₜ.
oder 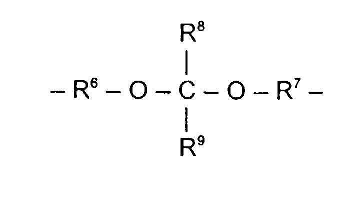
ist, worin:
s 1 bis 100 ist;
t 1 bis 12 ist;
R⁶ und R⁷ unabhängig ein C₁-C₁₂-Alkylen sind;
R⁸ Wasserstoff oder ein C₁-C₆-Alkyl ist; und
R⁹ ein C₁-C₆-Alkyl ist; oder
R⁸ und R⁹ zusammengenommen ein C₃-C₁₀-Alkylen sind;
R² 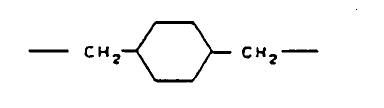 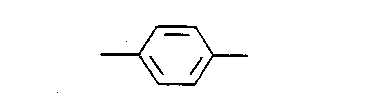 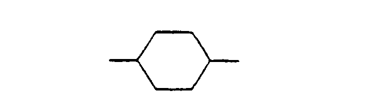 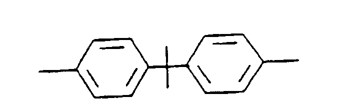 oder 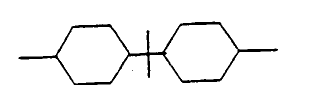
ist und in (-O-R³)_{q}- q 1 bis 20 ist;
und wenn q 1 ist, R³ 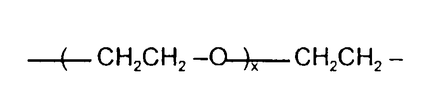 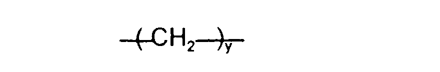 oder 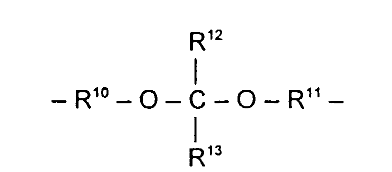 ist, worin:
x 1 bis 100 ist;
t 1 bis 12 ist;
R¹⁰ und R¹¹ unabhängig ein C₁-C₁₂-Alkylen sind;
R¹² Wasserstoff oder ein C₁-C₆-Alkyl ist; und
R¹³ ein C₁-C₆-Alkyl ist; oder
R¹² und R¹³ zusammengenommen ein C₃-C₁₀-Alkylen sind; und
wenn q 2 bis 20 ist, jedes R³ gleich oder verschieden sein kann und ausgewählt ist aus der Gruppe bestehend aus 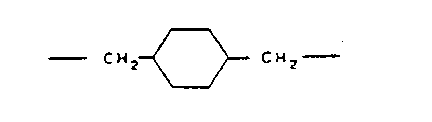 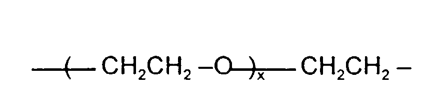 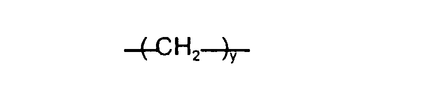 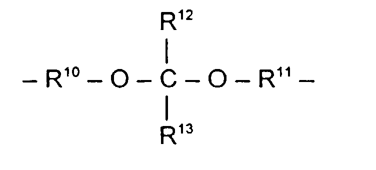 und 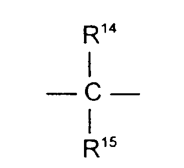
worin x, y, R¹⁰, R¹¹, R¹² und R¹³ wie oben definiert sind, R¹⁴ Wasserstoff oder ein C₁-C₄-Alkyl ist und R¹⁵ ein C₁-C₄-Alkyl ist, mit der Maßgabe, daß das Polymer mindestens 0,1 Mol-% der Einheit, in der A -O-R¹- ist, aufweist, worin das Verfahren umfaßt die Umsetzung eines Diketenacetals der Formel 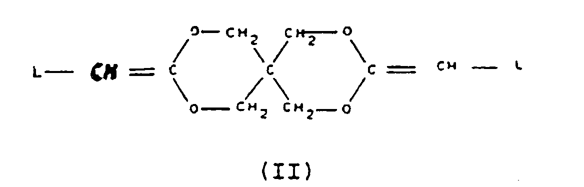 worin L Wasserstoff oder ein C₁₋₃-Alkyl ist; mit einem Diol der folgenden Formel lll(a) oder einer Mischung von zwei oder drei Diolen der folgenden Formeln lll(a), III(b) oder lll(c) in ausgewählten Verhältnissen:
HO-R¹-OH III(a)
HO-R²-OH III(b)
H(-O-R³)_{q}-OH III(c)
worin R¹, R², R³ und q wie vorstehend in der Präambel definiert sind, mit der Maßgabe, daß mindestens 0,1 Mol-% in der Diolmischung ein Diol der Formel lll(a) sind.

25. Verfahren nach Anspruch 24, worin eine Mischung von Diolen verwendet wird, die 1 bis 50 Mol-% des Diols der Formel lll(a) umfaßt.

26. Verfahren nach Anspruch 25, worin eine Mischung von Diolen verwendet wird, die 2 bis 30 Mol-% des Diols der Formel lll(a) umfaßt.

27. Verfahren nach Anspruch 24, worin eine Mischung von Diolen verwendet wird, die bis zu 20 Mol-% des Diols der Formel III(b) umfaßt.

28. Verfahren nach Anspruch 24, worin eine Mischung von Diolen verwendet wird, die 60 bis 99,9 Mol-% des Diols der Formel III(b) umfaßt.

29. Verfahren nach Anspruch 24, worin eine Mischung von Diolen verwendet wird, die ein Diol der Formel lll(a) umfaßt, worin R¹ 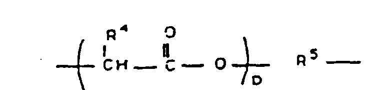 ist, worin R⁴ und R⁵ wie in Anspruch 24 definiert sind und p 1 bis 6 ist.

30. Verfahren nach Anspruch 29, worin p 1 bis 4 und bevorzugt 1 oder 2 ist.

31. Verfahren nach Anspruch 29 oder Anspruch 30, worin R⁴ Wasserstoff oder Methyl ist.

32. Verfahren nach Anspruch 31, worin R⁴ Wasserstoff oder Methyl ist und R⁵ 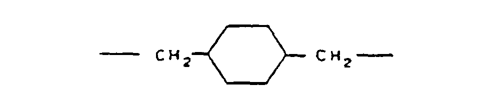 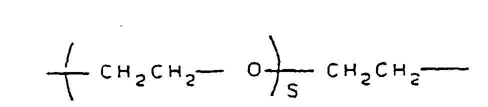 worin s 2 bis 6 ist,
oder
-(-CH₂-)ₜ-
worin t 4 bis 6 ist,
ist.

33. Verfahren nach Anspruch 32, worin s 2 ist und t 6 ist.

34. Verfahren nach Anspruch 32 oder Anspruch 33, worin R¹ im Diol der Formel lll(a) 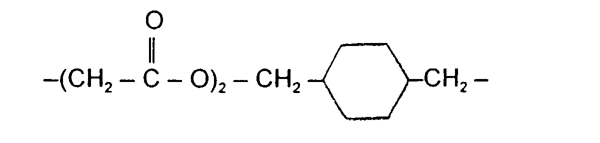 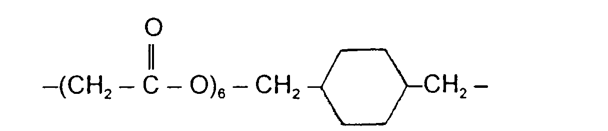 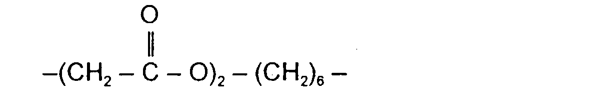 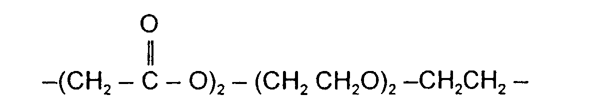 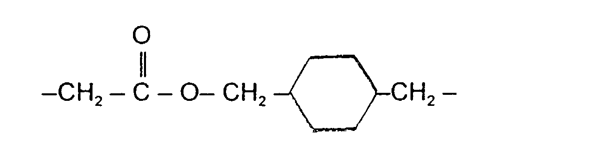 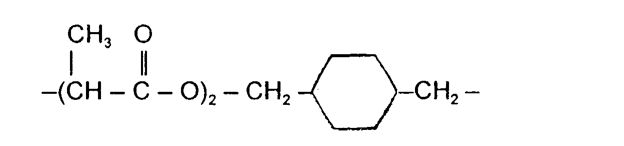 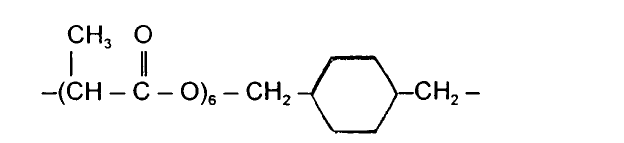 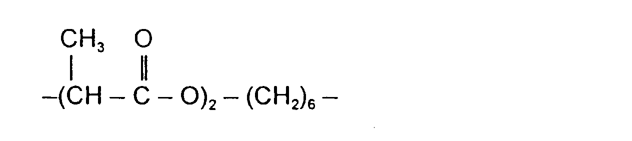 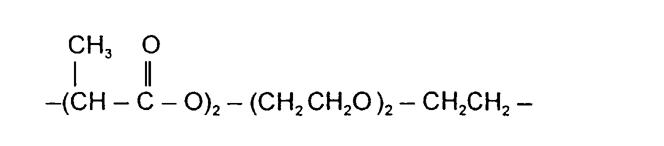 oder 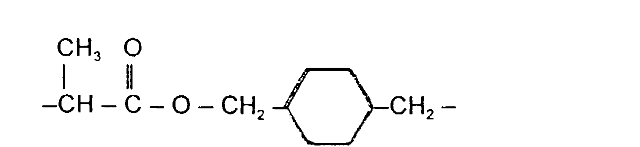 ist.

35. Verfahren nach Anspruch 31, worin R⁵ 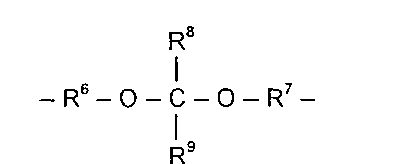 ist, worin R⁶ und R⁷ identisch sind und ein unverzweigtes C₄-C₁₂-Alkylen darstellen und R⁸ und R⁹ wie in Anspruch 24 definiert sind.

36. Verfahren nach Anspruch 35, worin R⁶ und R⁷ ein unverzweigtes C₆-C₁₂-Alkylen sind.

37. Verfahren nach Anspruch 35 oder Anspruch 36, worin R⁸ Wasserstoff und R⁹ Methyl ist.

38. Verfahren nach Anspruch 24, worin eine Mischung von Diolen verwendet wird, die ein Diol der Formel III(b) umfaßt, worin R² 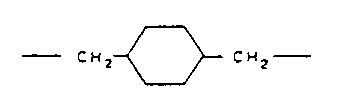 ist.

39. Verfahren nach Anspruch 24, worin eine Mischung von Diolen ein Diol der Formel lll(c) umfaßt, worin q 1 bis 6 ist und R³ wie in Anspruch 24 definiert ist.

40. Verfahren nach Anspruch 39, worin q 1 bis 3 ist.

41. Verfahren nach Anspruch 39, worin q 2 bis 6 ist und jedes R³ ausgewählt ist aus der Gruppe bestehend aus
-(-CH₂CH₂-O)ₓ-CH₂CH₂-
worin x 2 bis 6, bevorzugt 2, ist;
-(-CH₂-)_{y}-
worin y 4 bis 6, bevorzugt 6, ist; 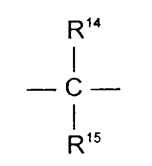 worin R¹⁴ Wasserstoff und R¹⁵ Methyl ist;
und

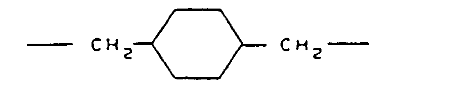

42. Verfahren nach Anspruch 41, worin (-O-R³)_{q}- 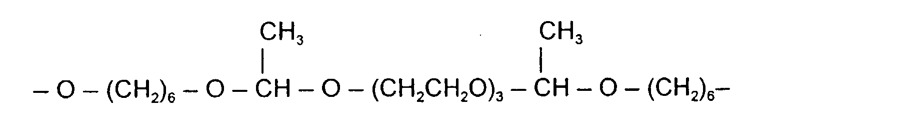 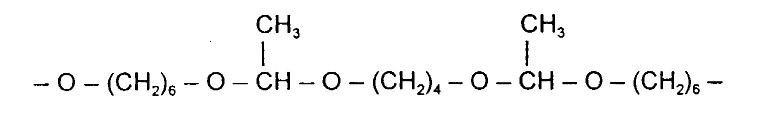 oder 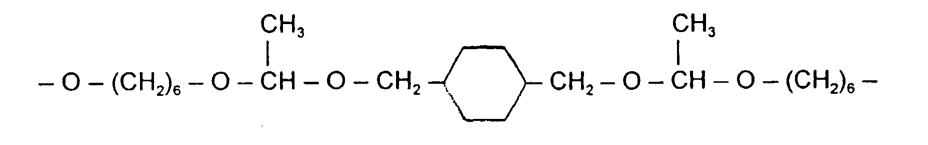 ist.

43. Verfahren nach Anspruch 39, worin q 1 ist und R³ 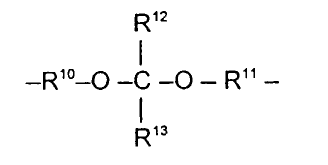 ist, worin R¹⁰ und R¹¹ beide ein unverzweigtes C₄-C₁₂-Alkylen sind und R¹² und R¹³ beide Methyl sind.

44. Verfahren nach Anspruch 43, worin R¹⁰ und R¹¹ ein unverzweigtes C₆-C₁₂-Alkylen sind.

45. Verfahren nach Anspruch 44, worin R¹⁰ und R¹¹ beide ein unverzweigtes C₁₀-Alkylen sind.

46. Verfahren nach irgendeinem der Ansprüche 24 bis 45, das eine Vernetzung mit einem Polyol, das mehr als zwei Hydroxygruppen enthält, beinhaltet.

47. Polymer, erhältlich durch Umsetzung zwischen einem Diketenacetal der Formel (II) mit einer Mischung von Diolen, die mindestens 0,1 Mol-% eines Diols der Formel lll(a), worin R¹ wie in irgendeinem der Ansprüche 29 bis 37 definiert ist, umfaßt und ferner entweder ein Diol der Formel lll(b), worin R² 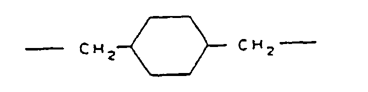 ist, oder ein Diol der Formel lll(c), worin R³ und q wie in irgendeinem der Ansprüche 39 bis 45 definiert sind, oder beide umfaßt.

48. Polymer, erhältlich durch Umsetzung zwischen
(a) einem Diketenacetal der Formel 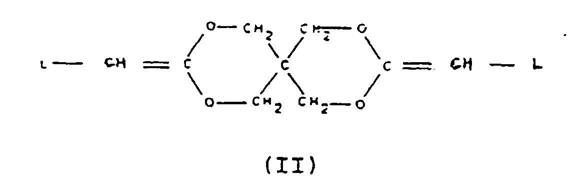 worin L Wasserstoff oder ein C₁₋₃-Alkyl ist; und
(b) mindestens einem Diol und mindestens einem Polyol mit mehr als 2 Hydroxygruppen, wobei mindestens 0,1 Mol-% davon ein α-Hydroxysäure enthaltendes Diol der Formel lll(a), wie es in irgendeinem der vorhergehenden Ansprüche definiert ist, sind.

## Revendications

1. Polymère de formule I 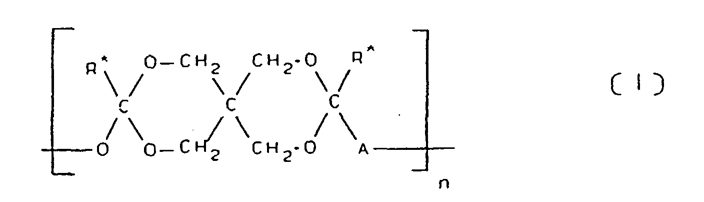 dans laquelle R* représente un groupe alkyle en C₁ à C₄, de préférence un groupe méthyle ou éthyle, A représente un groupe -O-R¹-, -O-R²- ou (-O-R³)_{q}-, n est ≥ 5 ; et
R¹ représente un groupe 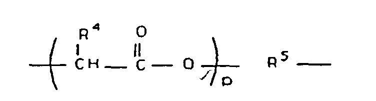
dans lequel :
p a une valeur de 1 à 10 ;
R⁴ représente l'hydrogène ou un groupe alkyle en C₁ à C₆ ; et
R⁵ représente un groupe 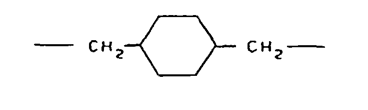 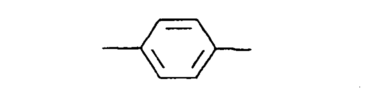 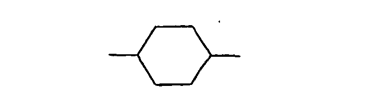 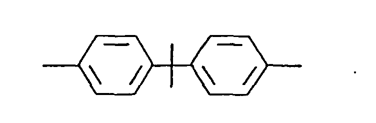 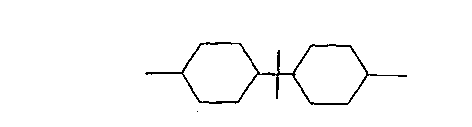 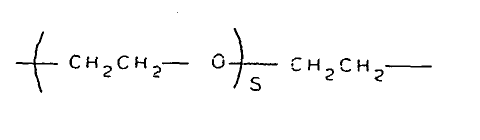
(̵CH₂)̵ₜ
ou 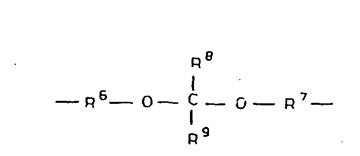
dans lequel :
s a une valeur de 1 à 100 ;
t a une valeur de 1 à 12 ;
R⁶ et R⁷ représentent indépendamment un groupe alkylène en C₁ à C₁₂ ;
R⁸ représente l'hydrogène ou un groupe alkyle en C₁ à C₆ ; et
R⁹ représente un groupe alkyle en C₁ à C₆ ; ou
R⁸ et R⁹ pris conjointement, représentent un groupe alkylène en C₃ à C₁₀ ; et
R² représente un groupe 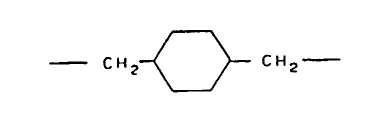 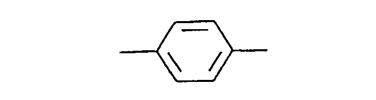 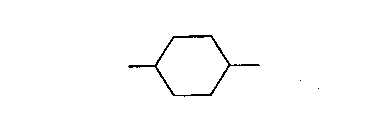 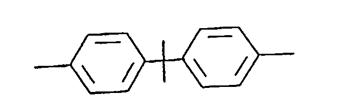 ou 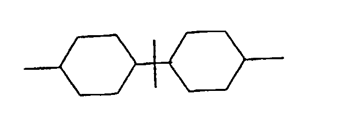
et, dans le groupe (-O-R³)_{q}-, q a une valeur de 1 à 20 ; lorsque q est égal à 1, R³ représente un groupe 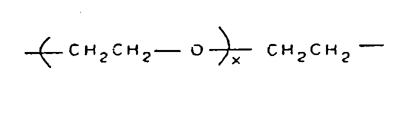 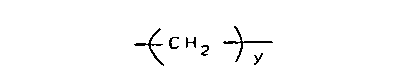 ou 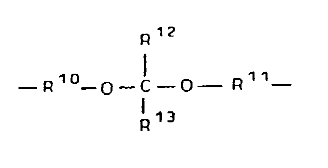 dans lequel :
x a une valeur de 1 à 100 ;
y a une valeur de 1 à 12 ;
R¹⁰ et R¹¹ représentent indépendamment un groupe alkylène en C₁ à C₁₂ ;
R¹² représente l'hydrogène ou un groupe alkyle en C₁ à C₆ ; et
R¹³ représente un groupe alkyle en C₁ à C₆ ; ou
R¹² et R¹³, pris conjointement, représentent un groupe alkylène en C₃ à C₁₀ ; et
lorsque q a une valeur de 2 à 20, les groupes R³ peuvent être identiques ou différents et sont choisis chacun dans le groupe consistant en des groupes 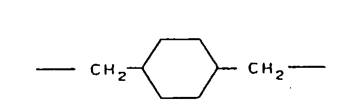 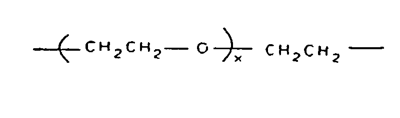 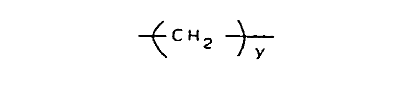 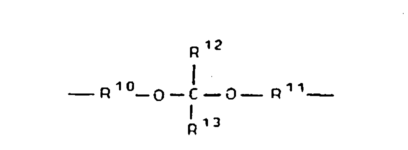 et 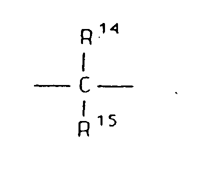 dans lesquels, x, y, R¹⁰, R¹¹, R¹² et R¹³ répondent aux définitions précitées, R¹⁴ représente l'hydrogène ou un groupe alkyle en C₁ à C₄, et R¹⁵ représente un groupe alkyle en C₁ à C₄, sous réserve que le polymère comprenne au moins 0,1 % en mole du motif dans lequel A représente un groupe -O-R¹-.

2. Polymère suivant la revendication 1, dans lequel n a une valeur d'environ 5 à environ 1000, avantageusement d'environ 20 à environ 500, de préférence d'environ 30 à environ 300.

3. Polymère suivant la revendication 1, comprenant environ 1 à environ 50 pour cent en mole du motif dans lequel A représente un groupe -O-R¹-.

4. Polymère suivant la revendication 3, comprenant environ 2 à environ 30 pour cent en mole du motif dans lequel A représente un groupe -O-R¹-.

5. Polymère suivant la revendication 1, dans lequel le motif dans lequel A est un groupe -O-R²- représente jusqu'à environ 20 pour cent en mole du polymère.

6. Polymère suivant la revendication 1, dans lequel le motif dans lequel A est un groupe -O-R²- représente environ 60 à environ 99,9 pour cent en mole du polymère.

7. Polymère suivant la revendication 1, comprenant le motif dans lequel A représente un groupe -O-R¹- et R¹ représente un groupe 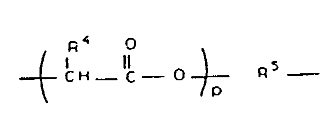 dans lequel R⁴ et R⁵ répondent aux définitions suivant la revendication 1 et p a une valeur de 1 à 6, avantageusement de 1 à 4 et de préférence la valeur 1 ou 2.

8. Polymère suivant la revendication 7, dans lequel R⁴ représente l'hydrogène ou un groupe méthyle.

9. Polymère suivant la revendication 8, dans lequel R⁴ représente l'hydrogène ou un groupe méthyle et R⁵ est choisi dans le groupe consistant en des groupes 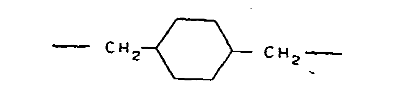 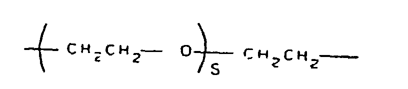 dans lesquels s a une valeur de 2 à 6 et est de préférence égal à 2 ;
et
(̵CH₂)̵ₜ
dans lequel t a une valeur de 4 à 6 et est de préférence égal à 6.

10. Polymère suivant la revendication 9, comprenant le motif dans lequel A représente un groupe -O-R¹- et R¹ représente un groupe 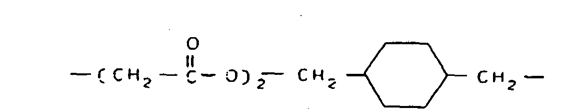 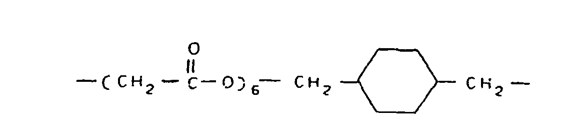 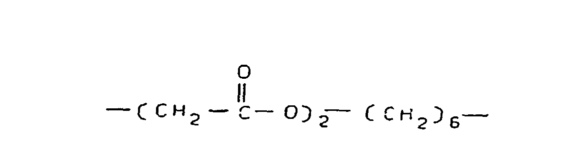 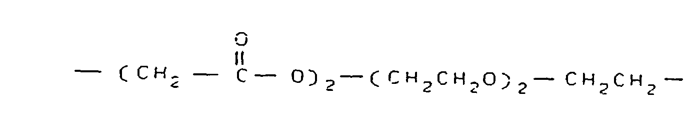 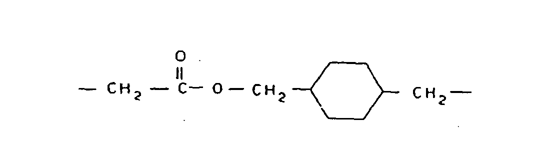 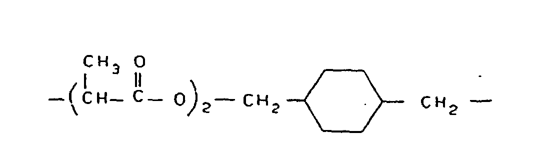 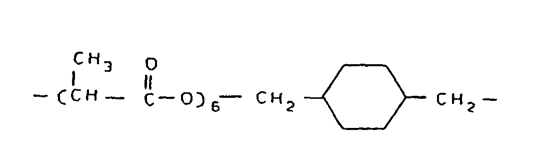 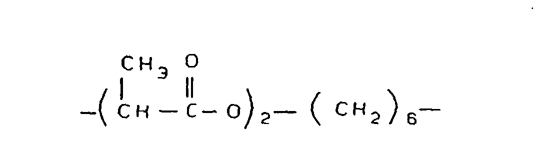 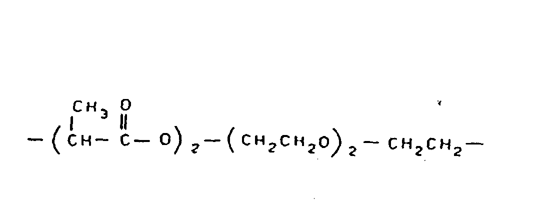 ou

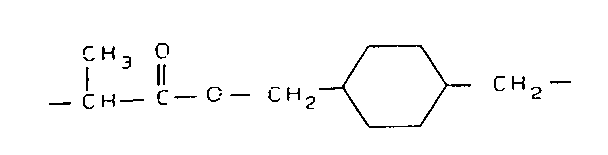

11. Polymère suivant la revendication 8, dans lequel R⁵ représente un groupe 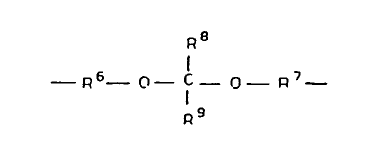 dans lequel R⁶ et R⁷ sont identiques et représentent un groupe alkylène en C₄ à C₁₂ non ramifié, de préférence un groupe alkylène en C₆ à C₁₂ non ramifié, et R⁸ et R⁹ répondent aux définitions suivant la revendication 1.

12. Polymère suivant la revendication 11, dans lequel R⁸ représente l'hydrogène et R⁹ représente un groupe méthyle.

13. Polymère suivant la revendication 1, comprenant le motif dans lequel A représente un groupe -O-R²- et R² représente un groupe

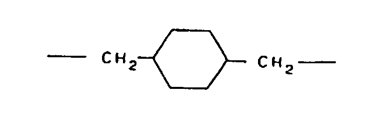

14. Polymère suivant la revendication 1, comprenant le motif dans lequel A représente un groupe (O-R³)_{q}- dans lequel q a une valeur de 1 à 6, de préférence de 1 à 3, et R³ répond à la définition suivant la revendication 1.

15. Polymère suivant la revendication 14, dans lequel q a une valeur de 2 à 6 et chaque groupe R³ est choisi dans le groupe consistant en des groupes 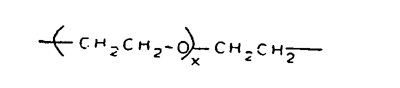 dans lequel x a une valeur de 2 à 6 et est de préférence égal à 2, 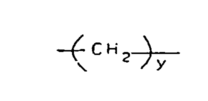 dans lequel y a une valeur de 4 à 6 et est de préférence égal à 6, 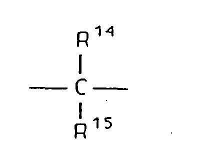 dans lequel R¹⁴ représente l'hydrogène et R¹⁵ représente un groupe méthyle,
et

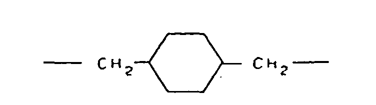

16. Polymère suivant la revendication 15, comprenant le motif dans lequel A représente un groupe (-O-R³)_{q}- et (-O-R³)_{q}- est un groupe 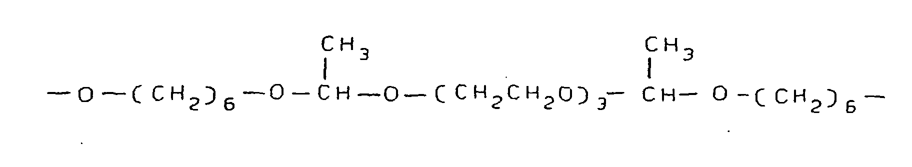 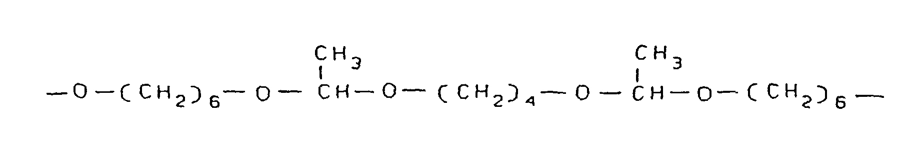 ou

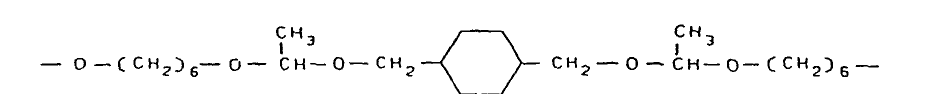

17. Polymère suivant la revendication 14, dans lequel q est égal à 1 et R³ représente un groupe 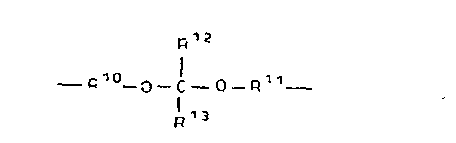 dans lequel R¹⁰ et R¹¹ représentent l'un et l'autre un groupe alkylène en C₄ à C₁₂ non ramifié, de préférence un groupe alkylène en C₆ à C₁₂ non ramifié, et R¹² et R¹³ représentent l'un et l'autre un groupe méthyle.

18. Polymère suivant la revendication 17, dans lequel R¹⁰ et R¹¹ représentent l'un et l'autre un groupe alkylène en C₁₀ non ramifié.

19. Polymère suivant les revendications 7 à 12, comprenant en outre facultativement le motif dans lequel A représente un groupe -O-R²- et R² représente un groupe 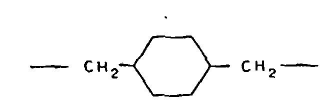 ou le motif dans lequel A représente un groupe (-O-R³)_{q}- et R³ et q répondent aux définitions suivant l'une quelconque des revendications 14 à 18, ou bien ces deux motifs.

20. Composition comprenant une matrice constituée d'un polymère suivant la revendication 1 et un agent actif.

21. Utilisation d'un polymère suivant la revendication 1 pour la préparation d'une composition destinée à la libération prolongée d'un agent actif.

22. Composition suivant la revendication 20 ou utilisation suivant la revendication 21, dans laquelle l'agent actif est un agent pharmaceutique, cosmétique ou agricole, de préférence un agent pharmaceutique tel qu'un antigène, un vaccin, un polypeptide, un agent hormonal, un agent antipsychotique ou un agent anti-néoplasique.

23. Polymère suivant la revendication 1, destiné à être utilisé en restauration orthopédique ou régénération tissulaire.

24. Procédé pour la préparation d'un polymère comprenant des motifs distincts répondant à la formule suivante 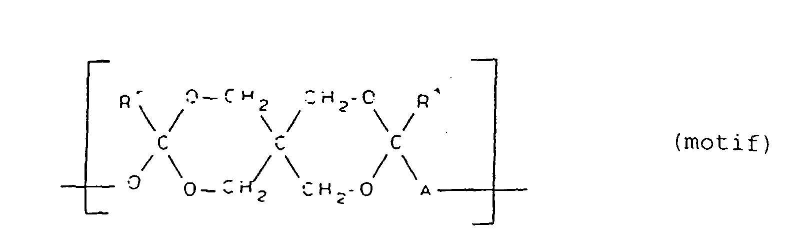 dans laquelle R* représente un groupe alkyle en C₁ à C₄, de préférence méthyle ou éthyle, A représente un groupe -O-R¹-, -O-R²- ou (-O-R³)_{q}- ; et
R¹ représente un groupe 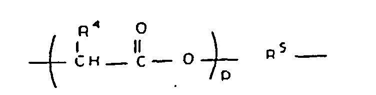
dans lequel :
p a une valeur de 1 à 10 ;
R⁴ représente l'hydrogène ou un groupe alkyle en C₁ à C₆ ; et
R⁵ représente un groupe 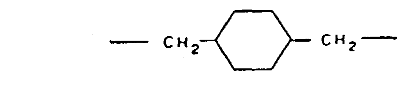 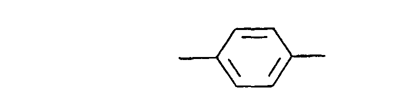 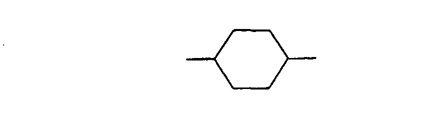 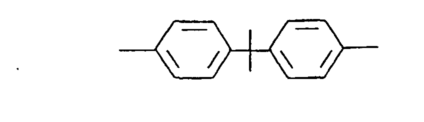 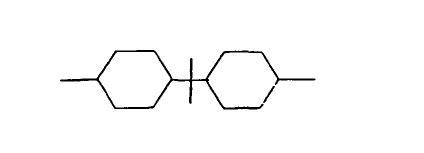 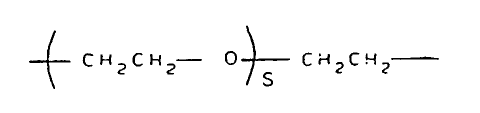
(̵CH₂)̵ₜ .
ou 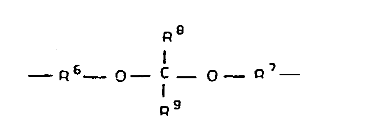
dans lequel :
s a une valeur de 1 à 100 ;
t a une valeur de 1 à 12 ;
R⁶ et R⁷ représentent indépendamment un groupe alkylène en C₁ à C₁₂ ;
R⁸ représente l'hydrogène ou un groupe alkyle en C₁ à C₆ ; et
R⁹ représente un groupe alkyle en C₁ à C₆ ; ou
R⁸ et R⁹ pris conjointement, représentent un groupe alkylène en C₃ à C₁₀ ;
R² représente un groupe 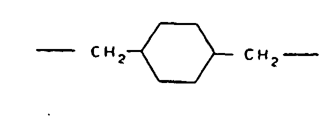 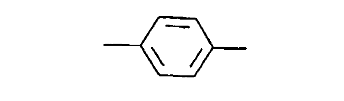 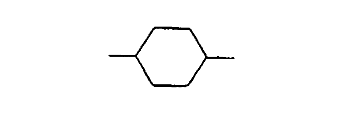 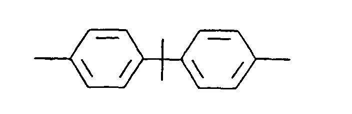 ou 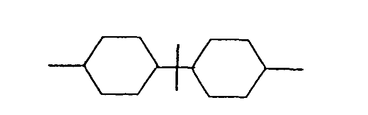
et, dans le groupe (-O-R³)_{q}-, q a une valeur de 1 à 20 ; lorsque q est égal à 1, R³ représente un groupe 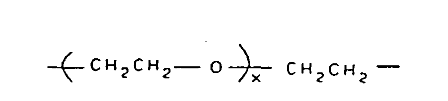 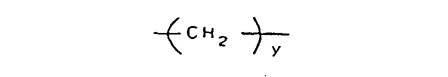 ou 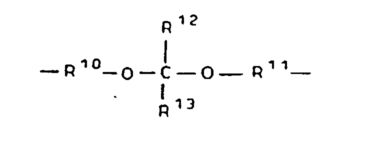 dans lequel :
x a une valeur de 1 à 100 ;
t a une valeur de 1 à 12 ;
R¹⁰ et R¹¹ représentent indépendamment un groupe alkylène en C₁ à C₁₂ ;
R¹² représente l'hydrogène ou un groupe alkyle en C₁ à C₆ ; et
R¹³ représente un groupe alkyle en C₁ à C₆ ; ou
R¹² et R¹³, pris conjointement, représentent un groupe alkylène en C₃ à C₁₀ ; et
lorsque q a une valeur de 2 à 20, les groupes R³ peuvent être identiques ou différents et sont choisis chacun dans le groupe consistant en des groupes 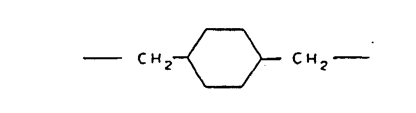 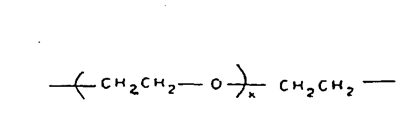 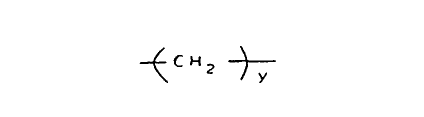 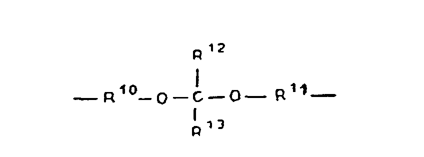 et 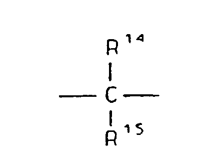 dans lesquels, x, y, R¹⁰, R¹¹, R¹² et R¹³ répondent aux définitions précitées, R¹⁴ représente l'hydrogène ou un groupe alkyle en C₁ à C₄, et R¹⁵ représente un groupe alkyle en C₁ à C₄, sous réserve que le polymère comprenne au moins 0,1 % en mole du motif dans lequel A représente un groupe -O-R¹-, procédé qui comprend la réaction d'un dicétène-acétal de formule 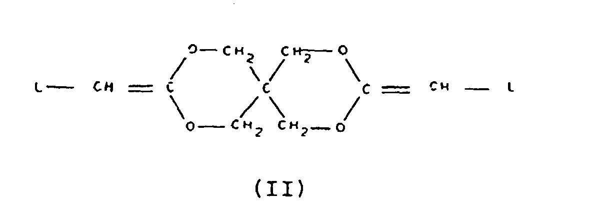 dans laquelle L représente l'hydrogène ou un groupe alkyle en C₁ à C₃, avec un diol répondant à la formule III(a) suivante ou un mélange de deux ou trois des diols répondant aux formules III(a), III(b) et III(c) suivantes en des proportions choisies :
HO-R¹-OH III(a)
HO-R²-OH III(b)
H(-O-R³)_{q}-OH III(c)
formules dans lesquelles R¹, R², R³ et q répondent aux définitions mentionnées ci-dessus dans le préambule, sous réserve qu'une quantité d'au moins 0,1 pour cent en mole dans le mélange de diols soit constituée d'un diol de formule III(a).

25. Procédé suivant la revendication 24, dans lequel un tel mélange de diols est utilisé et comprend 1 à 50 pour cent en mole du diol de formule III(a).

26. Procédé suivant la revendication 25, dans lequel un tel mélange de diols est utilisé et comprend 2 à 30 pour cent en mole du diol de formule III(a).

27. Procédé suivant la revendication 24, dans lequel un tel mélange de diols est utilisé et comprend jusqu'à 20 pour cent en mole du diol de formule III(b).

28. Procédé suivant la revendication 24, dans lequel un tel mélange de diols est utilisé et comprend 60 à 99,9 pour cent en mole du diol de formule III(b).

29. Procédé suivant la revendication 24, dans lequel un tel mélange de diols est utilisé et comprend un diol de formule III(a) dans laquelle R¹ représente un groupe 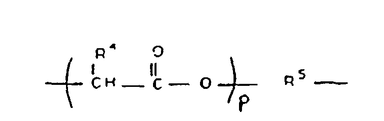 dans lequel R⁴ et R⁵ répondent aux définitions suivant la revendication 24 et p a une valeur de 1 à 6.

30. Procédé suivant la revendication 29, dans lequel p a une valeur de 1 à 4 et est de préférence égal à 1 ou 2.

31. Procédé suivant la revendication 29 ou la revendication 30, dans lequel R⁴ représente l'hydrogène ou un groupe méthyle.

32. Procédé suivant la revendication 31, dans lequel R⁴ représente l'hydrogène ou un groupe méthyle et R⁵ représente un groupe 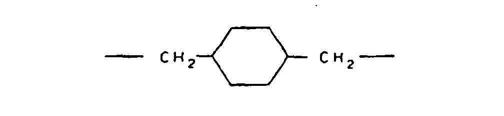 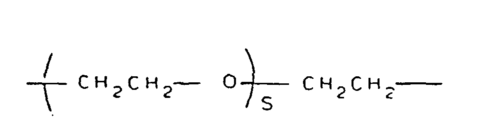 dans lequel s a une valeur de 2 à 6,
ou
(̵CH₂)̵ₜ
dans lequel t a une valeur de 4 à 6.

33. Procédé suivant la revendication 32, dans lequel s est égal à 2 et t est égal 6.

34. Procédé suivant la revendication 32 ou la revendication 33, dans lequel R¹ dans le diol de formule III(a) est un groupe 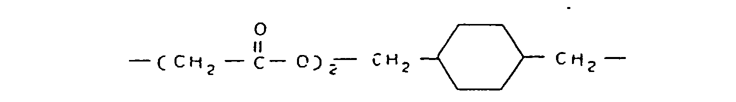 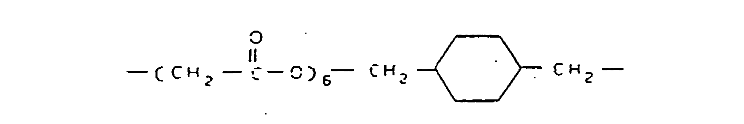 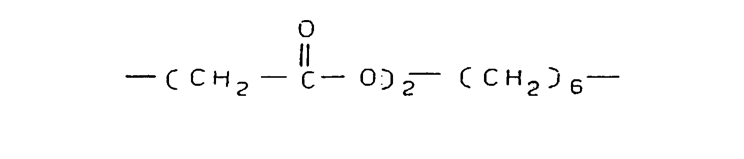 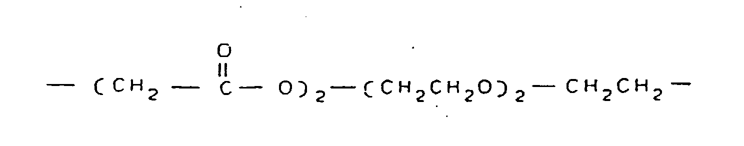 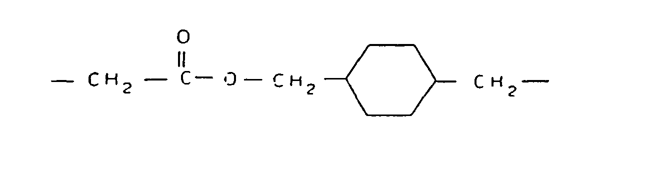 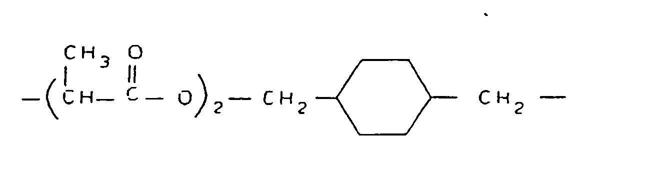 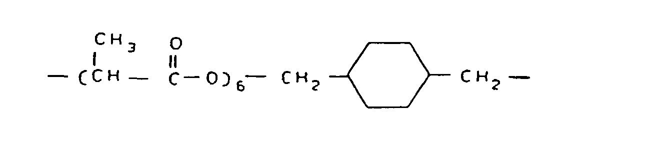 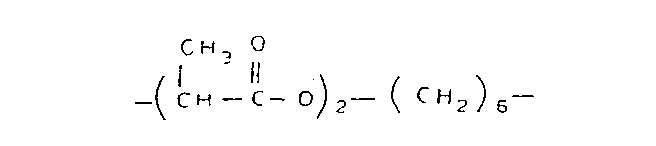 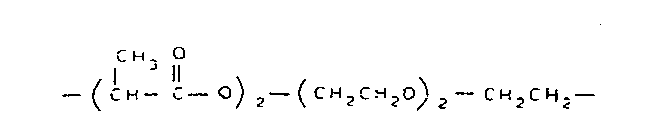 ou

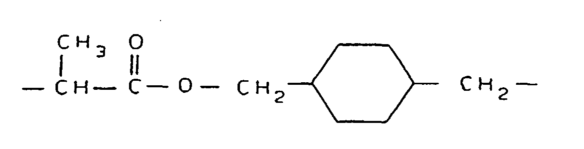

35. Procédé suivant la revendication 31, dans lequel R⁵ représente un groupe 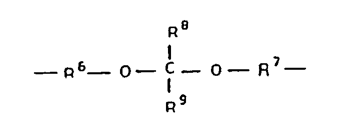 dans lequel R⁶ et R⁷ sont identiques et représentent un groupe alkylène en C₄ à C₁₂ non ramifié, et R⁸ et R⁹ répondent aux définitions suivant la revendication 24.

36. Procédé suivant la revendication 35, dans lequel R⁶ et R⁷ représentent un groupe alkylène en C₆ à C₁₂ non ramifié.

37. Procédé suivant la revendication 35 ou 36, dans lequel R⁸ représente l'hydrogène et R⁹ représente un groupe méthyle.

38. Procédé suivant la revendication 24, dans lequel un tel mélange de diols est utilisé et comprend un diol de formule III(b) dans laquelle R² représente un groupe

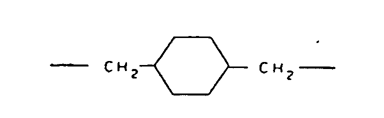

39. Procédé suivant la revendication 24, dans lequel le mélange de diols comprend un diol de formule III(c) dans laquelle q a une valeur de 1 à 6 et R³ répond à la définition suivant la revendication 24.

40. Procédé suivant la revendication 39, dans lequel q a une valeur de 1 à 3.

41. Procédé suivant la revendication 39, dans lequel q a une valeur de 2 à 6 et chaque groupe R³ est choisi dans le groupe consistant en des groupes 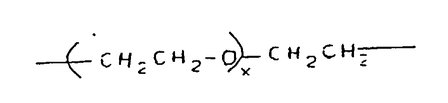 dans lequel x a une valeur de 2 à 6 et est de préférence égal à 2 ; 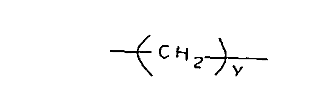 dans lequel y a une valeur de 4 à 6 et est de préférence égal à 6 ; 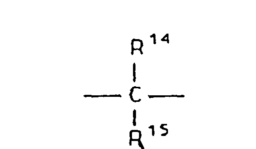 dans lequel R¹⁴ représente l'hydrogène et R¹⁵ représente un groupe méthyle ;
et

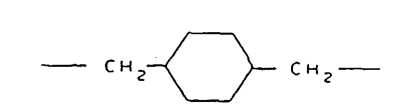

42. Procédé suivant la revendication 41, dans lequel le groupe (-O-R³)_{q}- est un groupe 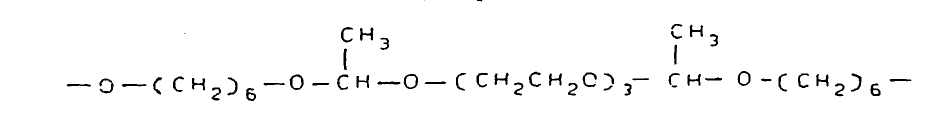 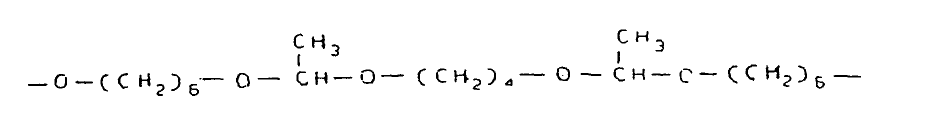 ou

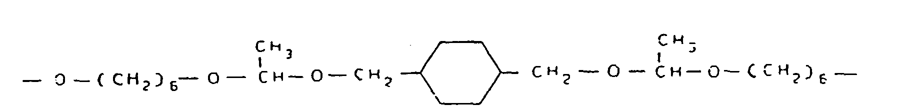

43. Procédé suivant la revendication 39, dans lequel q est égal à 1 et R³ représente un groupe 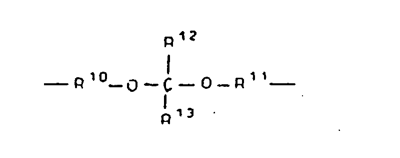 dans lequel R¹⁰ et R¹¹ représentent l'un et l'autre un groupe alkylène en C₄ à C₁₂ non ramifié, et R¹² et R¹³ représentent l'un et l'autre un groupe méthyle.

44. Procédé suivant la revendication 43, dans lequel R¹⁰ et R¹¹ représentent un groupe alkylène en C₆ à C₁₂ non ramifié.

45. Procédé suivant la revendication 44, dans lequel R¹⁰ et R¹¹ représentent l'un et l'autre un groupe alkylène en C₁₀ non ramifié.

46. Procédé suivant l'une quelconque des revendications 24 à 45, qui comprend une réticulation avec un polyol ne contenant pas plus de deux groupes hydroxy.

47. Polymère pouvant être obtenu par réaction entre un dicétène-acétal de formule (II) avec un mélange de diols comprenant au moins 0,1 pour cent en mole d'un diol de formule III(a) dans laquelle R¹ répond à la définition suivant l'une quelconque des revendications 29 à 37 et comprenant en outre un diol de formule III(b) dans laquelle R² représente un groupe 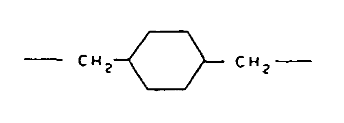 ou un diol de formule III(c) dans laquelle R³ et q répondent aux définitions suivant l'une quelconque des revendications 39 à 45, ou bien ces deux diols.

48. Polymère pouvant être obtenu par réaction entre :
(a) un dicétène-acétal de formule 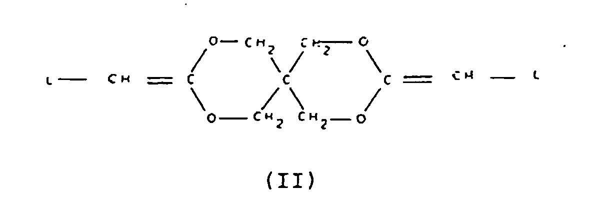 dans laquelle L représente l'hydrogène ou un groupe alkyle en C₁ à C₃ ; et
(b) au moins un diol et au moins un polyol ayant plus de deux groupes hydroxy, dont au moins 0,1 pour cent en mole consiste en un diol à fonction α-hydroxy-acide de formule III(a) répondant à la définition suivant l'une quelconque des revendications précédentes.
